(19) Europäisches Patentamt
European Patent Office
Office européen des brevets

(11) Veröffentlichungsnummer: **0 437 729 A2**

(12) # EUROPÄISCHE PATENTANMELDUNG

(21) Anmeldenummer: **90123781.8**

(22) Anmeldetag: **11.12.90**

(51) Int. Cl.[5]: **C07K 5/02**, A61K 37/64, C07D 213/75

(30) Priorität: **18.01.90 DE 4001236**

(43) Veröffentlichungstag der Anmeldung: **24.07.91 Patentblatt 91/30**

(84) Benannte Vertragsstaaten: **AT BE CH DE DK ES FR GB GR IT LI LU NL SE**

(71) Anmelder: **BAYER AG**
**W-5090 Leverkusen 1 Bayerwerk(DE)**

(72) Erfinder: **Voges, Klaus-Peter, Dr.**
**Schmitteborn 161**
**W-5600 Wuppertal 22(DE)**
Erfinder: **Häbich, Dieter, Dr.**
**Krummacher Strasse 82**
**W-5600 Wuppertal 1(DE)**
Erfinder: **Hansen, Jutta, Dr.**
**Claudiusweg 9**
**W-5600 Wuppertal 1(DE)**
Erfinder: **Paessens, Arnold, Dr.**
**Stresemannstrasse 51**
**W-5657 Haan(DE)**

(54) **Neue Peptide, Verfahren zu ihrer Herstellung und ihre Verwendung als Arzneimittel, insbesondere als Arzneimittel gegen Retroviren.**

(57) Die Erfindung betrifft neue Peptide, Verfahren zu ihrer Herstellung und ihre Verwendung als Arzneimittel, insbesondere als antivirale Mittel in der Human- und Tiermedizin.

EP 0 437 729 A2

## NEUE PEPTIDE, VERFAHREN ZU IHRER HERSTELLUNG UND IHRE VERWENDUNG ALS ARZNEIMITTEL, INSBESONDERE ALS ARZNEIMITTEL GEGEN RETROVIREN

Die Erfindung betrifft neue Peptide, Verfahren zu ihrer Herstellung und ihre Verwendung als Arzneimittel, insbesondere als antivirale Mittel in der Human- und Tiermedizin.

In den GB-A 22 03 740 und EP 33 77 14 werden Peptide beschrieben, die antivirale Aktivität gegen das "human immunodeficiency virus" (HIV) besitzen.

Ferner werden in der EP-A2 184 550 renininhibitorische Peptide beschrieben, in welcher die 2-Amino-2-methyl-propionsäure (AiB) über eine Sauerstoffbrücke mit der benachbarten Aminosäuregruppierung verknüpft ist.

Die vorliegende Erfindung betrifft neue Peptide der allgemeinen Formel (I)

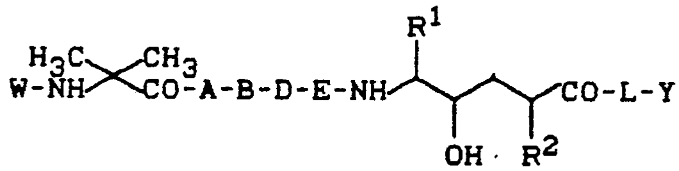

(I)

in welcher

W

- für eine Aminoschutzgruppe steht, oder
- für eine Gruppe der Formel

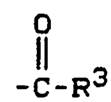

steht,

worin

$R^3$

- Wasserstoff, geradkettiges oder verzweigtes Alkyl mit bis zu 8 Kohlenstoffatomen oder Aryl mit 6 bis 10 Kohlenstoffatomen bedeutet, das seinerseits durch Halogen, Hydroxy, Nitro, Trifluormethyl oder durch geradkettiges oder verzweigtes Alkyl oder Alkoxy mit bis zu 8 Kohlenstoffatomen substituiert sein kann,

A, B, D, E und L gleich oder verschieden sind und

- für eine direkte Bindung oder
- für einen Rest der Formel

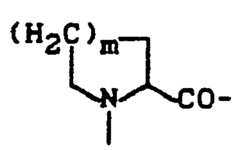

stehen

worin

m

- die Zahl 1 oder 2 bedeutet,

oder

- für eine Gruppe der Formel

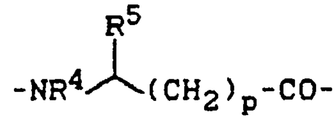

stehen
worin
p
- die Zahl 0, 1 oder 2 bedeutet,
$R^4$
- Wasserstoff, geradkettiges oder verzweigtes Alkyl mit bis zu 8 Kohlenstoffatomen oder Phenyl bedeutet,
$R^5$
- Cycloalkyl mit 3 bis 8 Kohlenstoffatomen oder Aryl mit 6 bis 10 Kohlenstoffatomen bedeutet, oder
- Wasserstoff oder geradkettiges oder verzweigtes Alkyl mit bis zu 8 Kohlenstoffatomen bedeutet, das gegebenenfalls durch Cycloalkyl mit 3 bis 8 Kohlenstoffatomen oder durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe $-NR^6R^7$ substituiert ist,
oder das gegebenenfalls durch einen 5- oder 6-gliedrigen stickstoffhaltigen Heterocyclus oder Indolyl substituiert ist, worin die entsprechenden -NH-Funktionen gegebenenfalls durch Alkyl mit bis zu 6 Kohlenstoffatomen oder durch eine Aminoschutzgruppe geschützt sind,
oder das gegebenenfalls durch Alkylthio mit bis zu 6 Kohlenstoffatomen, Hydroxy, Mercapto, Guanidyl oder durch eine Gruppe der Formel $-NR^6R^7$ oder $R^8$-OC- substituiert ist,
worin
$R^6$ und $R^7$
gleich oder verschieden sind und Wasserstoff, geradkettiges oder verzweigtes Alkyl mit bis zu 8 Kohlenstoffatomen oder Phenyl bedeuten
und
$R^8$
- Hydroxy, Benzyloxy, Alkoxy mit bis zu 6 Kohlenstoffatomen oder die oben aufgeführte Gruppe $-NR^6R^7$ bedeutet,

in ihrer D- oder L-Form, oder als D,L-Isomerengemisch, bevorzugt in der L-Form,
$R^1$ und $R^2$ gleich oder verschieden sind und
- für Cycloalkyl mit 3 bis 8 Kohlenstoffatomen stehen,
- für geradkettiges oder verzweigtes Alkyl oder Alkenyl mit bis zu 10 Kohlenstoffatomen stehen, die gegebenenfalls durch Cycloalkyl mit 3 bis 8 Kohlenstoffatomen oder Aryl mit 6 bis 10 Kohlenstoffatomen substituiert sind, das seinerseits bis zu 3-fach gleich oder verschieden durch Halogen, Cyano, Nitro oder durch eine Gruppe der Formel $-OR^9$ substituiert sein kann,
worin
$R^9$
- Wasserstoff oder eine typische Hydroxyschutzgruppe bedeutet,

Y
- für eine Gruppe der Formel $-NHR^{10}$ steht,
worin
$R^{10}$
Cycloalkyl mit 3 bis 8 Kohlenstoffatomen bedeutet, oder
- geradkettiges oder verzweigtes Alkyl mit bis zu 8 Kohlenstoffatomen bedeutet, das gegebenenfalls durch Pyridyl oder Phenyl substituiert ist

und deren physiologisch unbedenklichen Salze.

Aminoschutzgruppe im Rahmen der Erfindung sind die üblichen in der Peptid-Chemie verwendeten Aminoschutzgruppen.

Hierzu gehören bevorzugt: Benzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert-Butoxycarbonyl, 2-Nitrobenzyloxycarbonyl, 3,4,5-Trimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert-Butoxycarbonyl, Cyclohexoxycarbonyl, 1,1-Dimethylethoxycarbonyl, Adamantylcarbonyl, Phthaloyl, 2,2,2-Trichlorethoxycarbonyl, 2,2,2-Trichlor-tert-butoxycarbonyl, Menthyloxycarbonyl, Phenoxycarbonyl, 4-Nitrophenoxycarbonyl, Fluorenyl-9-methoxycarbonyl, Formyl, Acetyl, Propionyl, Pivaloyl, 2-Chloracetyl, 2-Bromacetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen,

Hydroxyschutzgruppe im Rahmen der Erfindung sind beispielsweise Trimethylsilyl, Triethylsilyl, Triisopropylsilyl, tert.Butyl-dimethylsilyl, tert.Butyl-diphenylsilyl, 2-Nitrobenzyl, Trifluormethoxy, Benzyl (Bzl), tert-Butyl ($^{t}$Bu), 2,2,2-Trichlorethyl (Tre), 4-Picolylether (Pic), Acetyl (Ac) oder 4-Toluolsulfonyl.

Die erfindungsgemäßen Verbindungen der allgemeinen Formel (I) haben mehrere asymmetrische Kohlenstoffatome. Sie können unabhängig voneinander in der D- oder der L-Form vorliegen. Die Erfindung umfaßt die optischen Antipoden ebenso wie die Isomerengemische oder Racemate. Bevorzugt liegen die Gruppen A, B, D, L und M unabhängig voneinander in der optisch reinen, bevorzugt in der L-Form vor.

Die allgemeine Formel Xa

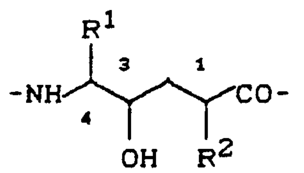

(Xa)

besitzt 3 asymmetrische Kohlenstoffatome (1, 3 und 4), die unabhängig voneinander in der R- oder S-Konfiguration vorliegen können. Bevorzugt liegt diese Gruppe in der 1R, 35, 45-Konfiguration , 1R, 3R, 4S-Konfiguration, 1S, 3R, 4S-Konfiguration oder in der 1S, 3S, 4S-Konfiguration vor. Besonders bevorzugt ist die 1S, 3S, 4S-Konfiguration.

Die erfindungsgemäßen Verbindungen der allgemeinen Formel (I) können in Form ihrer Salze vorliegen. Dies können Salze mit anorganischen oder organischen Säuren oder Basen sein. Zu den Säureadditionsprodukten gehören bevorzugt Salze mit Salzsäure, Bromwasserstoffsäure, Iodwasserstoffsäure, Schwefelsäure, Phosphorsäure oder mit Carbonsäuren wie Essigsäure, Propionsäure, Oxalsäure, Glykolsäure, Bernsteinsäure, Maleinsäure, Hydroxymaleinsäure, Methylmaleinsäure, Fumarsäure, Adipinsäure, Äpfelsäure, Weinsäure, Zitronensäure, Benzoesäure, Zimtsäure, Milchsäure, Ascorbinsäure, Salicylsäure, 2-Acetoxybenzoesäure, Nicotinsäure, Isonicotinsäure, oder Sulfonsäuren wie Methansulfonsäure, Ethansulfonsäure, Benzolsulfonsäure, Toluolsulfonsäure, Naphthalin-2-sulfonsäure oder Naphthalindisulfonsäure,

Bevorzugt sind Verbindungen der allgemeinen Formel (I),
in welcher
W
- für eine der oben aufgeführten Aminoschutzgruppen steht, vorzugsweise für tert.Butyloxycarbonyl (Boc), 9-Fluorenylmethyloxycarbonyl (Fmoc) oder Benzyloxycarbonyl (Z), oder
- für eine Gruppe der Formel

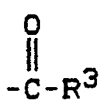

steht,
worin
$R^3$
- Wasserstoff, geradkettiges oder verzweigtes Alkyl mit bis zu 6 Kohlenstoffatomen oder Phenyl bedeutet, das seinerseits durch Fluor, Chlor, Brom oder durch geradkettiges oder verzweigtes Alkyl oder Alkoxy mit bis zu 6 Kohlenstoffatomen substituiert sein kann,

A, B, D, E und L gleich oder verschieden sind und
- für eine direkte Bindung oder
- für Prolin stehen, oder
- für eine Gruppe der Formel

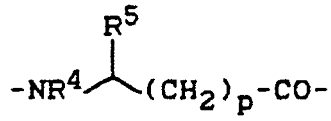

stehen
worin
p
die Zahl 0 oder 1 bedeutet,
$R^4$
- Wasserstoff oder geradkettiges oder verzweigtes Alkyl mit bis zu 6 Kohlenstoffatomen oder Phenyl bedeutet,

$R^5$
- Cyclopentyl oder Cyclohexyl bedeutet, oder Wasserstoff, Phenyl oder geradkettiges oder verzweigtes Alkyl mit bis zu 6 Kohlenstoffatomen bedeutet, das gegebenenfalls durch Hydroxy, HO-CO- oder $H_2N$-CO- substituiert sein kann,
oder durch Cyclohexyl oder Naphthyl oder Phenyl substituiert ist, die ihrerseits durch Fluor, Chlor, Nitro oder Alkoxy mit bis zu 6 Kohlenstoffatomen substituiert sein können,
oder durch Indolyl, Imidazolyl, Pyridyl, Triazolyl oder Pyrazolyl substituiert ist, wobei die entsprechenden -NH-Funktionen gegebenenfalls durch Alkyl mit bis zu 4 Kohlenstoffatomen oder durch eine Aminoschutzgruppe geschützt sind,

in ihrer D- oder L-Form, oder als D,L-Isomerengemisch, bevorzugt in ihrer L-Form,
$R^1$ und $R^2$ gleich oder verschieden sind und
- für Cyclopropyl, Cyclopentyl oder Cyclohexyl stehen,
- für geradkettiges oder verzweigtes Alkyl oder Alkenyl mit bis zu 8 Kohlenstoffatomen stehen, die gegebenenfalls durch Cyclopropyl, Cyclobutyl, Cyclopentyl, Cyclohexyl, Cycloheptyl oder Phenyl substiutiert sind, das seinerseits bis zu 2-fach gleich oder verschieden durch Fluor, Chlor, Brom, Cyano, Nitro oder durch eine Gruppe der Formel $-OR^9$ substiutiert ist,
worin
$R^9$
- Wasserstoff, tert-Butyl oder Benzyl bedeutet,

Y
- für eine Gruppe der Formel $-NHR^{10}$ steht,
worin
$R^{10}$
geradkettiges oder verzweigtes Alkyl mit bis zu 6 Kohlenstoffatomen bedeutet, das gegebenenfalls durch Cyclopropyl, Cyclopentyl, Cyclohexyl, Phenyl oder Pyridyl substituiert ist

und deren physiologisch unbedenklichen Salze.

Besonders bevorzugt sind Verbindungen der allgemeinen Formel (I),
in welcher
W
- für die Aminoschutzgruppen Boc, FMoc oder Benzyloxycarbonyl steht, oder
- für eine Gruppe der Formel

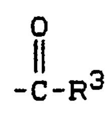

steht,
worin
$R^3$
- Wasserstoff, geradkettiges oder verzweigtes Alkyl mit bis zu 4 Kohlenstoffatomen oder Phenyl bedeutet,

A, B, D, E und L gleich oder verschieden sind und
- für eine direkte Bindung oder
- für Prolin stehen, oder

- für eine Gruppe der Formel

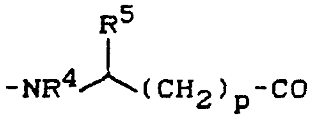

stehen,
worin
p
die Zahl 0 oder 1 bedeutet,
$R^4$
- Wasserstoff oder Methyl bedeutet,
$R^5$
- Cyclopentyl oder geradkettiges oder verzweigtes Alkyl mit bis zu 4 Kohlenstoffatomen bedeuten, das gegebenenfalls durch Hydroxy, HO-CO- oder $H_2N$-CO- substituiert ist, oder durch Cyclohexyl oder Naphthyl oder Phenyl substituiert ist, die ihrerseits durch Fluor, Chlor oder Alkoxy mit bis zu 4 Kohlenstoffatomen substiuiert sein können, oder durch Imidazolyl, Triazolyl, Pyridyl oder Pyrazolyl substituiert ist, wobei die NH-Funktion gegebenenfalls durch Methyl, Benzyloxymethylen oder t-Butyloxycarbonyl (Boc) geschützt sind,

in ihrer D- oder L-Form oder als D,L-Isomerengemisch, bevorzugt in der L-Form,
$R^1$ und $R^2$ gleich oder verschieden sind und
- für Cyclopropyl, Cyclopentyl oder Cyclohexyl stehen, oder
- für geradkettiges oder verzweigtes Alkyl oder Alkenyl mit bis zu 6 Kohlenstoffatomen stehen, die gegebenenfalls durch Cyclopropyl, Cyclopentyl, Cyclohexyl oder Phenyl substituiert sind, das seinerseits durch eine Gruppe der Formel $-OR^9$ substituiert ist,
worin
$R^9$
- Wasserstoff, tert.Butyl oder Benzyl bedeutet,

Y
- für eine Gruppe der Formel $-NHR^{10}$ steht,
worin
$R^{10}$
geradkettiges oder verzweigtes Alkyl mit bis zu 4 Kohlenstoffatomen bedeutet, das gegebenenfalls durch Cyclopropyl, Cyclohexyl, Phenyl oder Pyridyl substituiert ist

und deren physiologisch unbedenklichen Salze.

Außerdem wurde ein Verfahren zur Herstellung der Verbindungen der allgemeinen Formel (I) gefunden, dadurch gekennzeichnet, daß man
Verbindungen der allgemeinen Formel (II)

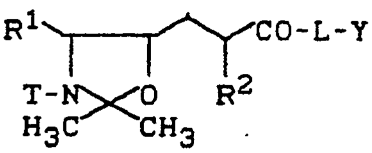

(II)

in welcher
$R^1$, $R^2$, L und Y die oben angegebene Bedeutung haben
und
T
- für eine der oben aufgeführten Aminoschutzgruppen, vorzugsweise für Benzyloxycarbonyl steht,

in inerten Lösemitteln zu den Aminoalkoholen der Formel (III)

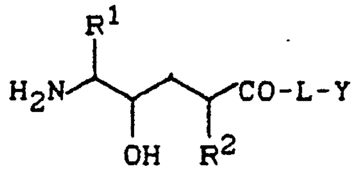

(III)

in welcher

$R^1$, $R^2$, L und Y die oben angegebene Bedeutung haben,

unter Ringöffnung reduziert, und anschließend mit Verbindungen der Formel (IV)

T'-E'-OH (IV)

in welcher

T'

- die oben angegebene Bedeutung von T hat und mit dieser gleich oder verschieden ist

und

E'

die oben angegebene Bedeutung von E hat, aber nicht für eine direkte Bindung steht,

nach üblicher Methode kondensiert,

in einem nächsten Schritt, entweder zunächst die Schutzgruppe T' abspaltet, erneut deblockiert und mit Verbindungen der allgemeinen Formel (V),

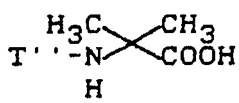

(V)

in welcher

T"

die oben angegebene Bedeutung von T hat und mit dieser gleich oder verschieden ist,

gegebenenfalls unter Aktivierung der Carbonsäure nach üblicher Methode zu Verbindungen der allgemeinen Formel (Ia)

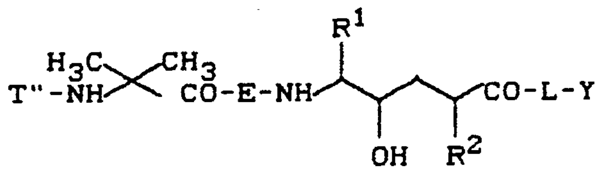

(Ia)

in welcher

T", E, $R^1$, $R^2$, L und Y die oben angegebene Bedeutung haben,

umsetzt oder zunächst die Aminosäuregruppierungen B und D in Analogie zu der oben beschriebenen Umsetzung mit den Verbindungen der Formel (IV) unter Aktivierung und Deblockierung einführt und in einem letzten Schritt mit Verbindungen der allgemeinen Formel (VI)

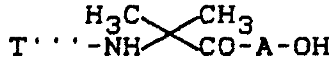

(VI)

in welcher

A die oben angegebene Bedeutung hat

und

T'''

die oben angegebene Bedeutung von T hat und mit dieser gleich oder verschieden ist,

umsetzt.

Das erfindungsgemäße Verfahren kann beispielhaft durch folgendes Formelschema erläutert werden:

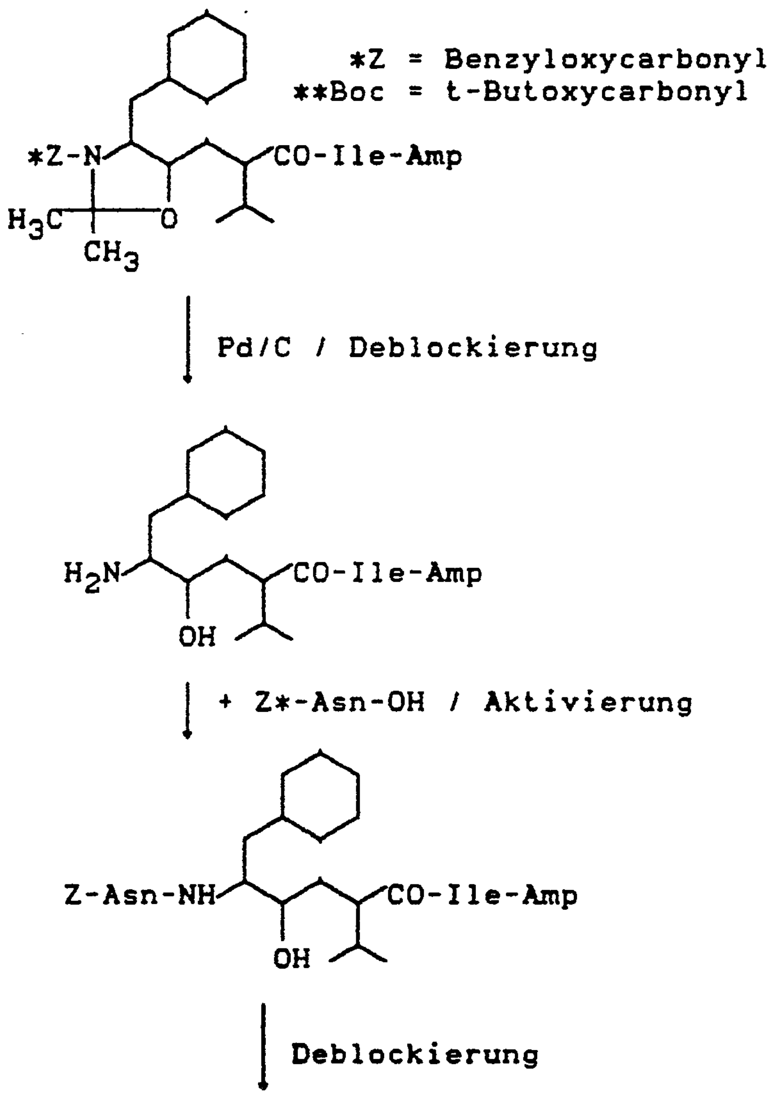
*Z = Benzyloxycarbonyl
**Boc = t-Butoxycarbonyl
Pd/C / Deblockierung
+ Z*-Asn-OH / Aktivierung
Deblockierung

a)

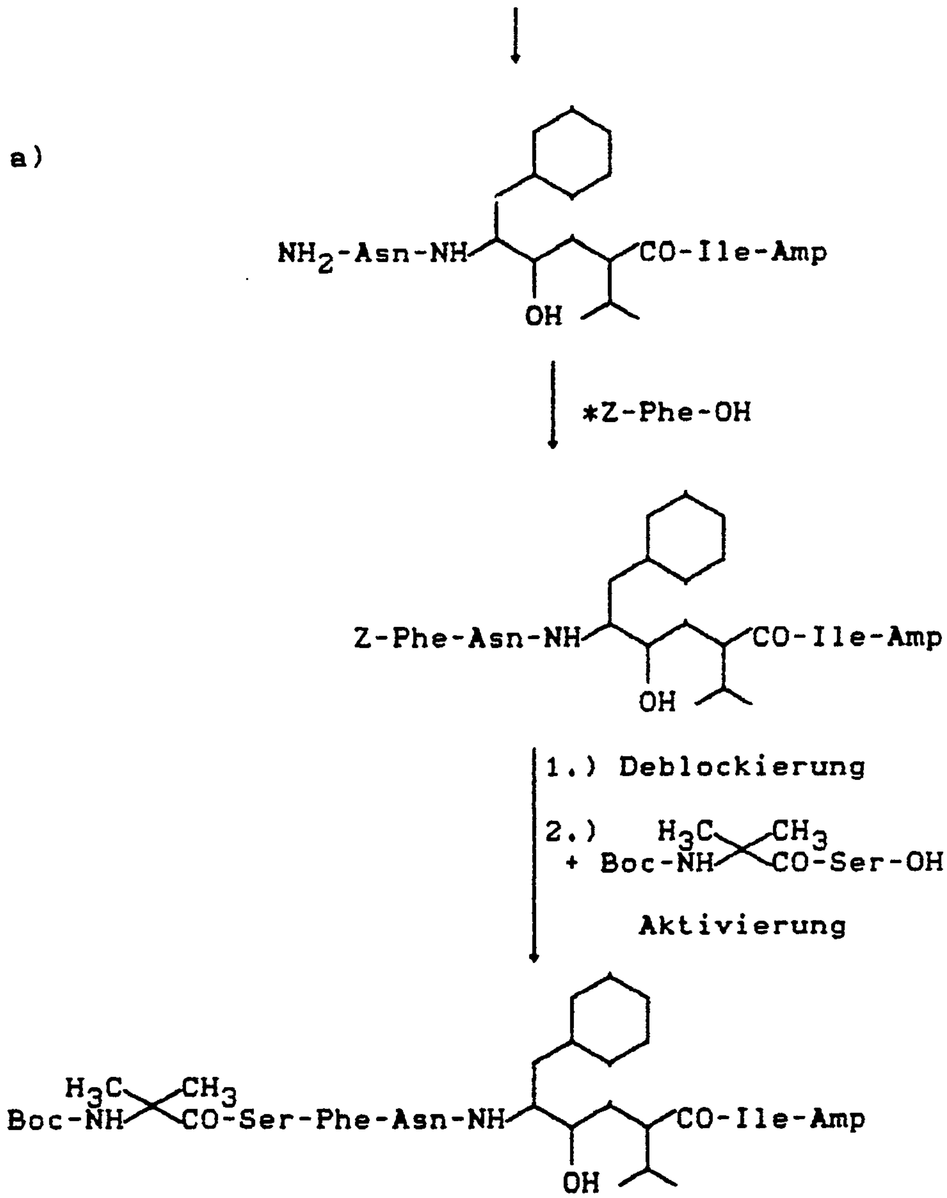

b)

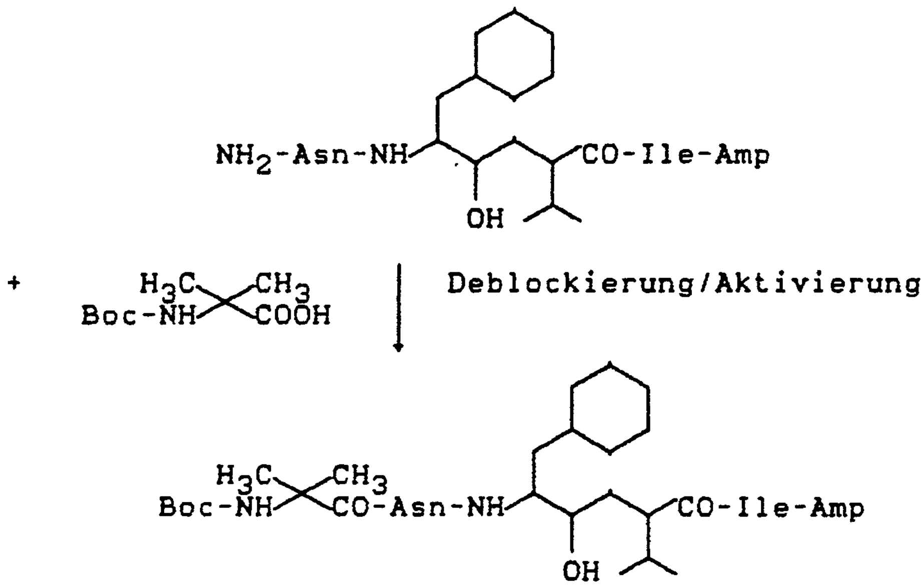


Als Lösemittel eignen sich für alle Verfahrensschritte die üblichen inerten Lösemittel, die sich unter den Reaktionsbedingungen nicht verändern. Hierzu gehören bevorzugt organische Lösemittel wie Ether z,B. Diethylether, Glykolmono-oder dimethylether, Dioxan oder Tetrahydrofuran, oder Kohlenwasserstoffe wie Benzol, Toluol, Xylol, Cyclohexan oder Erdölfraktionen oder Halogenkohlenwasserstoffe wie Methylenchlorid, Chloroform, Tetrachlorkohlenwasserstoff, oder Dimethylsulfoxid, Dimethylformamid, Hexamethylphosphorsäuretriamid, Essigester, Pyridin, Acetonitril, Triethylamin oder Picoline. Ebenso ist es möglich, Gemische der genannten Lösemittel zu verwenden.

Besonders bevorzugt sind für die Reduktion Methanol und Essigsäureethylester.

Die Reduktion der Verbindungen der allgemeinen Formel (II) erfolgt entweder mit den üblichen Katalysatoren, wie beispielsweise Palladiumhydroxid oder Palladium/Kohlenstoff, vorzugsweise mit Palladium/Kohlenstoff oder über eine katalytishe Transferhydrierung in an sich bekannter Weise [vgl. Tetrahedron 41, 3469 (1985), 3463 (1985), Synthesis 1987, 53].

Der Katalysator wird in einer Menge von 0,01 bis 0,5 mol, bevorzugt von 0,02 bis 0,05 mol zugesetzt, bezogen auf 1 Mol der Verbindung der allgemeinen Formel (II).

Die Reduktion wird in einem Temperaturbereich von 40° C bis 160° C, vorzugsweise von 80° C bis 100° C durchgeführt.

Die Reduktion kann sowohl bei Normaldruck als auch bei erhöhtem oder erniedrigtem Druck (beispielsweise 0,5 bis 5 bar), vorzugsweise bei Normaldruck durchgeführt werden.

Die Verbindungen der allgemeinen Formel (II) sind an sich bekannt oder können nach üblicher Methode hergestellt werden [vgl. EP-A2 143 746].

Die Verbindungen der allgemeinen Formel (III) sind an sich bekannt [vgl. beispielsweise EP-A2 236 734, US 475 8584, EP-A2 143 746].

Die Verbindungen der allgemeinen Formel (IV) sind an sich bekannt oder können nach üblicher Methode hergestellt werden [vgl. Houben-Weyl, Methoden der organischen Chemie, Synthese von Peptiden II, 4. Aufl. Bd. 15/1, 15/2, Georg Thieme Verlag, Stuttgart].

Die Umsetzung mit den Verbindungen der allgemeinen Formel (IV) und die Einführung der Aminosäuregruppierung A, B, D und L erfolgt im allgemeinen durch Umsetzung eines entsprechenden Bruchstückes, bestehend aus einer oder mehreren Aminosäuregruppierungen, mit einer freien, gegebenenfalls in aktivierter Form vorliegenden Carboxylgruppe mit einem komplementierenden Bruchstück, bestehend aus einer oder mehreren Aminosäuregruppierungen, mit einer Aminogruppe, gegebenenfalls in aktivierter Form, und indem man diesen Vorgang gegebenenfalls so oft mit entsprechenden Bruchstücken wiederholt, bis man die gewünschten Peptide der oben angegebenen allgemeinen Formeln hergestellt hat, anschließend gegebenenfalls Schutzgruppen abspaltet oder gegen anderen Schutzgruppen austauscht.

Als Hilfsstoffe für die jeweiligen Peptidkupplungen werden bevorzugt Kondensationsmittel eingesetzt, die auch Basen sein können, insbesondere wenn die Carboxylgruppe als Anhydrid aktiviert vorliegt. Bevorzugt werden hier die üblichen Kondensationsmittel wie Carbodiimide z.B. N,N'.-Diethyl-, N,N'-Dipropyl-, N,N'-Diisopropyl-, N,N'-Dicyclohexylcarbodiimid, N-(3-Dimethylaminoisopropyl)-N'-ethylcarbodiimid-Hydrochlorid, N-Cyclohexyl-N'-(2-morpholinoethyl)-carbodiimid-metho-p-toluolsulfonat,

oder Carbonylverbindungen wie Carbonyldiimidazol, oder 1,2-Oxazoliumverbindungen wie 2-Ethyl-5-phenyl-1,2-oxazolium-3-sulfatoder 2-tert-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Benzotriazolyloxy-tris(dimethylamino)phosphoniumhexafluorophosphat oder 1-Hydroxybenzotriazol.

Außerdem können beispielsweise Alkalicarbonate z,B, Natrium- oder Kaliumcarbonat oder -hydrogencarbonat, oder organische Basen wie Trialkylamine z.B. Triethylamin, N-Ethylmorpholin, N-Methylpiperidin oder N-Methylmorpholin eingesetzt werden. Bevorzugt ist Triethylamin.

Die Hilfsstoffe und Basen werden in einer Menge von 1,0 Mol bis 3,0 Mol, bevorzugt 1,0 bis 1,2 Mol, bezogen auf jeweils 1 Mol der Verbindungen der allgemeinen Formel (IV) oder (V) eingesetzt.

Die Peptidkupplungen werden in einem Temperaturbereich von 0°C bis 100°C, vorzugsweise bei 10 bis 50°C und bei Normaldruck durchgeführt.

Die Reaktionen können sowohl bei Normaldruck als auch bei erhöhtem oder erniedrigtem Druck (beispielsweise 0,5 bis 5 bar), vorzugsweise bei Normaldruck durchgeführt werden.

Die Abspaltung der jeweiligen Schutzgruppen vor den einzelnen Peptidknüpfungen erfolgt in an sich bekannter Weise unter sauren oder basischen Bedingungen, oder reduktiv durch katalytische Hydrierung beispielsweise mit Pd/C in organischen Lösemitteln wie Ethern, z.B. Tetrahydrofuran oder Dioxan, oder Alkoholen z.B. Methanol, Ethanol oder Isopropanol [vg]. Protective Groups in Organic Synthesis, W. Greene, John Wiley & Sons, New York, 1981; Chemistry and Biochemistry of the Amino Acids, G.C. Barrett, Chapman and Hall, London, New York, 1985].

Die Verbindungen der allgemeinen Formeln (V) und (VI) sind bekannt oder können nach üblicher Methode hergestellt werden.

Es wurde überraschend gefunden, daß die Verbindungen der allgemeinen Formel (I) eine außerordentlich starke Wirkung gegen Retroviren besitzen. Dies wird mit einem HIV-spezifischen Protease-Enzymtest belegt.

Die Ergebnisse der unten aufgeführten Beispiele wurden nach dem in den folgenden Literaturangaben [vg]. Hansen, J., Billich, S., Schulze, T., Sukrow, S. and Mölling, K. (1988), EMBO Journal, Vol, 7, No. 6, pp. 1785-1791] beschriebenen HIV-Testsystem ermittelt: Gereinigte HIV-Protease wurde mit synthetischem Peptid, das eine Schnittstelle im Gag-Precursor-Protein imitiert und eine in vivo-Spaltstelle der HIV-Protease darstellt, inkubiert. Die entstandenen Spaltprodukte des synthetischen Peptids wurden über Reverse Phase High Performance Liquid Chromatography (RP-HPLC) analysiert. Die angegebenen $IC_{50}$-Werte beziehen sich auf die Substanzkonzentration, die unter den oben aufgeführten Testbedingungen eine 50%ige Hemmung der Protease-Aktivität bewirkt.

| Beispiel-Nr. | $IC_{50}$ (RP-HPLC) |
|---|---|
| I | $5 \times 10^{-7}$ M |
| II | $5 \times 10^{-8}$ M |
| III | $5 \times 10^{-9}$ M |
| IV | $10^{-7}$ M |
| V | $5 \times 10^{-8}$ M |
| VIa | $10^{-9}$ M |
| VIb | $10^{-9}$ M |
| VIIa | $10^{-9}$ M |

Die erfindungsgemäßen Verbindungen zeigen außerdem eine starke antivirale Wirkung in der HIV-I infizierten Zellkultur an frisch präparierten menschlichen Blutlymphozyten (PBL's). Der HIV-Test wurde mit geringen Modifikationen nach der Methode von Panwels et al. (Journal of Virological Methods 20 (1988) 309-321) durchgeführt.

| Beispiel Nr. | $IC_{50}$* (ug/ml) |
|---|---|
| VIa | 0,40 |
| VIb | 0,09 |
| VIIa | 1,00 |
| VIIb | 3,00 |

*$IC_{50}$ = 50 % inhibitorische Konzentration

Es wurde gefunden, daß die erfindungsgemäßen Verbindungen mit HIV-Virus infizierten Zellen vor der virusinduzierten Zellzerstörung schützen.

Die erfindungsgemäßen Verbindungen stellen wertvolle Wirkstoffe zur Behandlung und Prophylaxe von Erkrankungen, hervorgerufen durch Retroviren, in der Human-und Tiermedizin dar.

Als Indikationsgebiete in der Humanmedizin können beispielsweise genannt werden:

1.) Die Behandlung oder Prophylaxe von menschlichen Retrovirusinfektionen.

2.) Für die Behandlung oder Prophylaxe von HIV I (Virus der humanen Immundefizienz; früher HTLV III/LAV genannt) und durch HIV II verursachten Erkrankungen (AIDS) und den damit assozierten Stadien wie ARC (AIDS related complex) und LAS (Lymphadenopathie-Syndrom) sowie der durch dieses Virus verursachten Immunschwäche und Encephalopathie.

3.) Für die Behandlung oder die Prophylaxe einer HTLV I-oder HTLV II-Infektion.

4.) Für die Behandlung oder die Prophylaxe des AIDS-carrier Zustandes (AIDS-Überträger-Zustand).

Als Indikationen in der Tiermedizin können beispielsweise angeführt werden:

Infektionen mit

a) Maedivisna (bei Schafen und Ziegen)

b) progressivem Pneumonievirus (PPV) (bei Schafen und Ziegen)

c) caprine arthritis encephalitis Virus (bei Schafen und Ziegen)

d) Zwoegerziekte Virus (bei Schafen)

e) infektiösem Virus der Anämie (des Pferdes)

f) Infektionen verursacht durch das Katzenleukämievirus

g) Infektionen verursacht durch das Virus der Katzen-Immundefizienz

Bevorzugt werden aus dem Indikationsgebiet in der Humanmedizin die oben aufgeführten Punkte 2, 3 und 4.

Zur vorliegenden Erfindung gehören pharmazeutische Zubereitungen, die neben nicht-toxischen, inerten pharmazeutisch geeigneten Trägerstoffen eine oder mehrere Verbindungen der Formel (I) enthalten oder die aus einem oder mehreren Wirkstoffen der Formel (I) bestehen, sowie Verfahren zur Herstellung dieser Zubereitungen.

Die Wirkstoffe der Formel (I) sollen in den oben aufgeführten pharmazeutischen Zubereitungen in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den Verbindungen der Formel (I) auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich sowohl in der Human- als auch in der Veterinärmedizin als vorteilhaft erwiesen, den oder die Wirkstoffe der Formel (I) in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

Anhang zum experimentellen Teil

I. Liste der verwendeten Dünnschichtsysteme:

| | | |
|---|---|---|
| Ia | $CH_2Cl_2/CH_3OH$ | 90:10 |
| Ib | $CH_2Cl_2/CH_3OH$ | 95:5 |
| Ic | $CH_2Cl_2/CH_3OH$ | 85:15 |
| II | $CH_2Cl_2$ | |
| IIIa | $CH_2Cl_2/CH_3OH/CH_3COOH/H_2O$ | 65:25:3:4 |
| IIIb | $CH_2Cl_2/CH_3OH/H_2O$ | 65:25:4 |
| IV | $CH_2Cl_2/CH_3OH/CH_3COOH$ | 90:10:0,1 |
| V | Petrolether(Sdp. 60-90$^0$ C)/Diethylether | 1:1 |
| VI | Toluol/Ethylacetat | 4:1 |
| VII | Toluol/Ethylacetat | 6:1 |
| VIII | Toluol/Ethylacetat | 2:1 |
| IX | Hexan/Ethylacetat | 4:1 |

II. Aminosäuren

Im allgemeinen erfolgt die Bezeichnung der Konfiguration durch das Vorausstellen eines L bzw. D vor der Aminosäureabkürzung, im Fall des Racemats durch ein D,L-wobei zur Vereinfachung bei L-Aminosäuren die Konfigurationsbezeichnung unterbleiben kann und dann nur im Fall der D-Form bzw, des D,L-Gemisches explizierte Bezeichnung erfolgt.

| | |
|---|---|
| Ala | L-Alanin |
| Arg | L-Arginin |
| Asn | L-Asparagin |
| Asp | L-Asparaginsäure |
| Cys | L-Cystein |
| Gln | L-Glutamin |
| Glu | L-Glutaminsäure |
| Gly | L-Glycin |
| His | L-Histidin |
| Ile | L-Isoleucin |
| Leu | L-Leucin |
| Lys | L-Lysin |
| Phe | Phenylalanin |
| Ser | Serin |

III. Aktivierungsreagenzien

| | |
|---|---|
| HOBT | 1-Hydroxybenzotriazol |
| HOSU | N-Hydroxysuccinimid |
| DCC | Dicyclohexylcarbodiimid |
| Morpho-CDI | N-Cyclohexyl-N'-(2-morpholinoethyl)-carbodiimid-metho-p-toluolsulfonat |
| BOP | Benzotriazolyloxy-tris(dimethylamino)phosphoniumhexaphosphate |

IV. Schutzgruppen (Hydroxyl, Amino)

| | |
|---|---|
| Bzl | Benzyl |
| tBu | tert.Butyl |
| Boc | tert.Butylcarbonyl |
| Z | Benzyloxycarbonyl |
| Fmoc | 9-Fluorenylmethoxycarbonyl |
| AMP | 2-Aminomethylpyridin |
| AEP | 2-Aminoethylpyridin |
| BOM | Benzyloxymethylen |

## Ausgangsverbindungen

### Beispiel 1

N-(Benzyloxycarbonyl)-2(S)-amino-3-cyclohexylpropionsäure

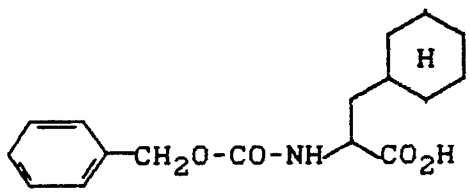

290 g (1,7 mol) 2-Amino-3-cyclohexylpropionsäure werden in 375 ml 4 N NaOH gelöst. Die Lösung wird auf 2 1 mit Wasser aufgefüllt und mit 500 ml Dioxan versetzt. Unter pH-Stat-Bedingungen werden 255 ml (1,7 mol) Benzyloxycarbonylchlorid bei pH 10 zugetropft. Nach 8 h wird die Lösung zweimal mit Diethylether extrahiert und mit 1N Salzsäure bis auf pH 2 angesäuert. Das Produkt wird 3 mal mit je 200 ml Ethylacetat extrahiert. Nach Waschen des organischen Extraktes, Trocknen über Natriumsulfat und Einengen erhält man 5 g der Titelverbindung.
DC: $R_f$ (IIIa) = 0.81 (TDM)
MS (EI): 305
SF(MG): $C_{17}H_{23}NO_4$ (305,374)
TDM = N,N,N',N'-Tetramethyl-4,4'-diaminodiphenylmethan

### Beispiel 2

Methyl-N-methyl-[2-(S)-(Benzyloxycarbonyl)-amino-3-cyclohexylpropionsäure]hydroxamat

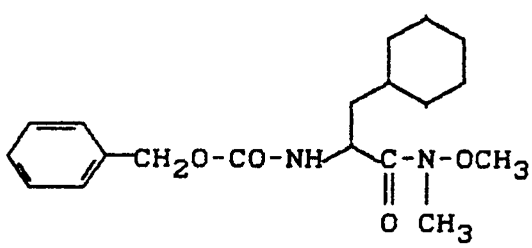

305 g (1 mol) der Verbindung aus Beispiel 1 und 107,2 g (1,1 mol) N,O-Dimethylhydroxylaminhydrochlorid werden in 2000 ml Dichlormethan gelöst und bei 0° C mit 725 ml (6 mol) N-Methylpiperidin versetzt. Bei -20° C werden 590 ml (1,2 mol) n-Propylphosphorsäureanhydrid zugetropft. Nach 12 h Rühren bei Raumtemperatur wird eingeengt und der Rückstand gegen Natriumbicarbonat-Lösung mit Ethylacetat verteilt. Die organische Phase wird einmal mit Kaliumhydrogensulfat und zweimal mit Kochsalzlösung gewaschen. Nach Trocknen über Natriumsulfat und Einengen erhält man 240 g (69% der Theorie) der Titelverbindung.
DC: $R_f$ (Ib) = 0,67 (TDM)
MS (DCI) = 349
SF(MG): $C_{19}H_{28}N_2O_4$ (348,21)

$^1$H-NMR (DMSO-$D_6$): $\delta$ = 0,7 - 1,8 (m, 13H); 3,1 (s, 3H); 3,7 (s, 3H); 4,55 (t, 1H); 5,0 (s, 2H); 7,3 (m, 5H); 7,55 (d, 1H) ppm.

Beispiel 3

2(S)-(Benzyloxycarbonyl)amino-3-cyclohexylpropanal

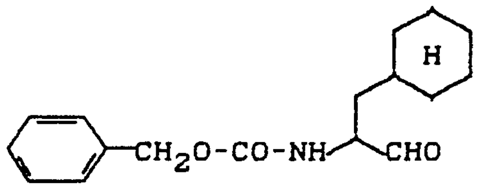

Unter Stickstoff werden 230 g (0,66 mol) der Verbindung aus Beispiel 2 in 1000 ml getrocknetem Diethylether gelöst und bei 0 °C tropfenweise mit 780 ml einer 1N Lösung von Lithiumaluminiumhydrid in Diethylether versetzt. Nach beendeter Zugabe wird 45 Minuten bei 0 °C nachgerührt und dann vorsichtig mit einer 5%igen Lösung von Kaliumhydrogensulfat angesäuert. Die wäßrige Phase wird abgetrennt und zweimal mit Diethylether rückextrahiert. Die vereinigten organischen Phasen werden 3 mal mit 0,5 M Kaliumhydrogensulfatlösung, 3 mal mit gesättigter Natriumhydrogencarbonatlösung und 2 mal mit gesättigter Kochsalzlösung gewaschen. Nach Trocknen über Natriumsulfat und Einengen erhält man 193 g der Titelverbindung in leicht verunreinigter Form. Die Verbindung muß bei -20 °C gelagert und möglichst sofort weiter umgesetzt werden.

DC: $R_f$ (Ib) = 0,36 (TDM, 2,4-Dinitrophenylhydrazin)
MS (EI) = 289
IR = 1710, 2850, 2900 $cm^{-1}$
SF(MG): $C_{17}H_{23}NO_3$ (289,18)
$^1$H-NMR (DMSO-$d_6$): $\delta$ = 9,5 (s, 1H) ppm.

Beispiel 4

2'(S)-(Benzyloxycarbonyl)amino-but-3'-en-yl-cyclohexan

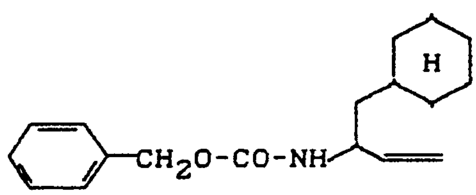

274,6 g (0,66 mol) Instant-Ylid® werden in 500 ml absolutem Tetrahydrofuran 30 Minuten lang gerührt. 190,86 g (0,66 mol) der Verbindung aus Beispiel 3 werden in 400 ml absolutem Tetrahydrofuran gelöst und unter Eiskühlung zu der Ylid-Suspension getropft. Man läßt bei Raumtemperatur 12 h nachrühren. Die Suspension wird auf 1000 ml Eis gegossen und 4 mal mit Petrolether (Sdp 60-90 °C) extrahiert. Nach Waschen der organischen Phase mit Kochsalzlösung und Trocknen über Magnesiumsulfat erhält man 190 g der Titelverbindung.

DC: $R_f$ (II) = 0,49 (TDM, $I_2$)
DC: $R_f$ (Ia) = 0,93
MS (DCI) = 288
SF(MG): $C_{18}H_{25}NO_2$ (287,19)
$^1$H-NMR (DMSO-$d_6$): $\delta$ = 0,8 - 1,8 (m, 13H); 4,05 (t, 1H); 5,0 (d+m, 4H); 5,7 (m, 1H); 7,3 (m, 5 + 1H) ppm.

Beispiel 5 und Beispiel 6

2(R)-{1(S)-[1-(Benzoxycarbonyl)amino-2-cyclohexyl]-ethyl}oxiran (Beispiel 5)

2(S)-{1(S)-[1-(Benzoxycarbonyl)amino-2-cyclohexyl]-ethyl}oxiran (Beispiel 6)

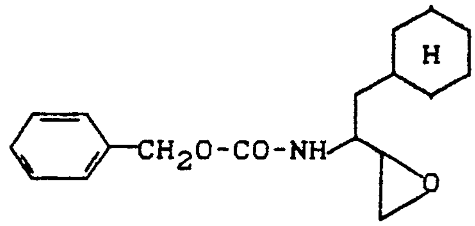

189,55 g (0,66 mol) der Verbindung aus Beispiel 4 werden in 600 ml Dichlormethan gelöst und bei 0 °C mit 227,79 g (1,32 mol) 3-Chlorperoxybenzoesäure portionsweise versetzt. Nach 12 h Rühren wird die Lösung 3 mal mit gesättigter Natriumsulfatlösung und 3 mal mit gesättigter Natriumcarbonatlösung gewaschen. Nach Trocknen und Einengen erhält man 278,3 g eines Öls. Das Rohprodukt wird an Kieselgel chromatographiert [Säule 30 x 10 cm, Petrolether (Sdp. 60-90 °C) : Diethylether (2:1)]. Die erste Fraktion erhält 3-Chlorbenzoesäure.

In Fraktion 2 werden 52,04 g der Titelverbindung erhalten.

DC: $R^f$ (V) = 0,53

MS (DCI) = 304

SF(MG): $C_{18}H_{25}NO_3$ (303,2)

$^1$H-NMR ($CDCl_3$): $\delta$ = 0,8 - 1,9 (m, 13H); 2,55 (t, 1H); 2,7 (t, 1H); 2,95 (s, 1H); 4,0 (q, 1H); 4,7 (d, 1H); 5,1 (s, 2H); 7,3 (m, 5H) ppm.

36,82 g der Titelverbindung (Beispiel 6) werden als Fraktion 3 der Säulenchromatographie erhalten.

DC: $R_f$ (V) = 0,45

MS (DCI) = 304

SF: $C_{18}H_{25}NO_3$

Beispiel 7

3(S)-(Benzyloxycarbonyl)amino-4-cyclohexyl-2(S)-hydroxy-1-iodobutan

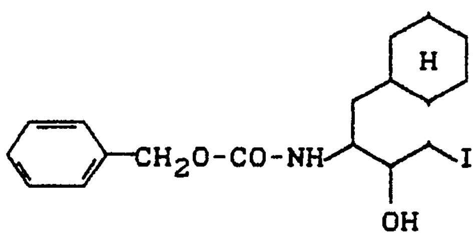

52,04 g (172 mmol) der Verbindung aus Beispiel 5 werden in 400 ml Acetonitril gelöst und bei 0 °C 25,82 g (172 mmol) Natriumjodid versetzt. Bei 0 °C werden 22,3 ml (172 mmol) Trimethylchlorsilan innerhalb von 30 Minuten zugetropft. Man läßt 1 h bei 0 °C nachrühren. Die Suspension wird auf 1000 ml Eiswasser gegossen und 3 mal mit je 200 ml Diethylether extrahiert. Nach Waschen der organischen Phase mit 0,1 M Natriumthiosulfatlösung und Kochsalzlöung wird über Natriumsulfat getrocknet und eingeengt. Das Rohprodukt (66,8 g eines Öls) wird über eine Flashsäule chromatographiert (20 x 10 cm, Dichlormethan). Als Nebenprodukt wird das O-$Si(CH_3)_3$-Derivat erhalten, das durch Behandeln mit wässriger Kaliumfluoridlösung in die Titelverbindung gespalten werden kann.

Man erhält 57,5 g eines farblosen Öls.

DC: $R_f$ (Ib) = 0,60

DC: $R_f$ (VI) = 0,49

MS (EI) = 431

SF(MG): $C_{18}H_{26}NO_3I$ (431,1)

Beispiel 8

3-Benzyloxycarbonyl-4(S)-cyclohexylmethyl-2,2-dimethyl-5(S)-iodomethyl-oxazolidin

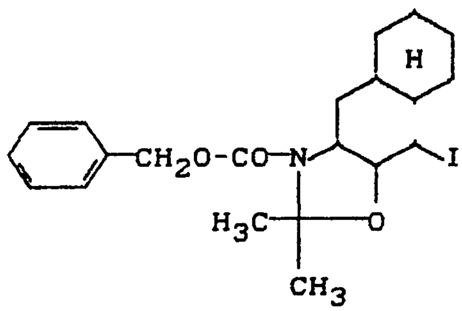

57,6 g (113 mmol) der Verbindung aus Beispiel 7 werden in 230 ml Dimethylformamid gelöst. Nach Zugabe von 200 mg (1.16 mmol) para-Toluolsulfonsäure werden 19,2 g (266 mmol) 2-Methoxypropen zugetropft und die Lösung 12h auf 80°C erhitzt. Nach dem Abkühlen wird der Rückstand in einer 1:1-Mischung aus Petrolether (Sdp. 60-90°C) und Dichlormethan aufgenommen und mit gesättigter Natriumhydrogencarbonatlösung 3 mal flashchromatographiert [400 g Kieselgel, Petrolether (Sdp. 60-90°C) : Dichlormethan (1:1)]. Man erhält 54,7 g eines weißen Feststoffes.

DC: $R_f$ (IX) = 0,53 (TDM, $I_2$)

Smp.: Substanz ist wachshaltig

MS (FAB) = 472

SF(MG): $C_{21}H_{30}NO_3I$ (471,38)

$^1$H-NMR (DMSO-$d_6$): $\delta$ = 0,8 - 1,8 (m, 13H); 1,3 (s, 3H); 1,4 (s, 3H); 3,3 (t, 1H); 3,45 (t, 1H); 3,95 (m, 1H); 4,05 (m, 1H); 5,1 (q, 2H); 7,3 (m, 5H) ppm.

Beispiel 9

3-Benzyloxycarbonyl-4(S)-cyclohexylmethyl-2,2-dimethyl-5(S)-[1'-(2',2'-bismethoxycarbonyl)ethyl]oxazolidin

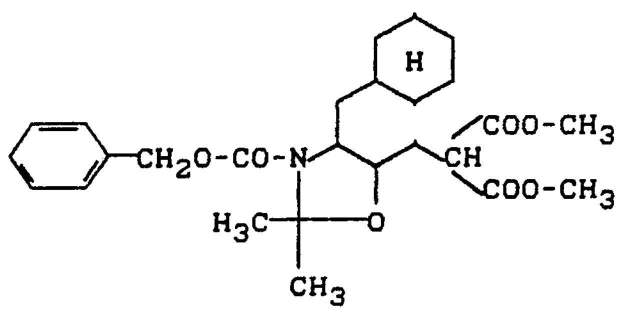

Unter Stickstoff werden 11,96 ml (104,4 mmol) Malonsäuredimethylester in 160 ml getrocknetem Dimethoxyethan verrührt und mit 3,13 g (104,4 mmol) Natriumhydrid (80%ig) versetzt. Nach 10 Minuten bei Raumtemperatur werden 41,1 g (87 mmol) der Verbindung aus Beispiel 8 zugegeben. Nach 12 h unter Rückfluß (140°C) wird abgekühlt und überschüssiges Hydrid mit 1N Zitronensäure gequenscht. Nach dem Einengen wird der Rückstand in Essigester aufgenommen, 3 mal mit Wasser gewaschen, über Natriumsulfat getrocknet und eingeengt. Man erhält 38,2 g eines Öle. Chromatographie an Kieselgel (Toluol/Essigester 100:0 → 50:50) ergibt 27,64 g der Titelverbindung in Fraktion 2. Als Fraktion 1 werden 3 g Ausgangsmaterial zurückgewonnen.

DC: $R_f$ (VII) = 0,48

DC: Rf (VIII) = 0,34

MS (DCI): 476,2

SF(MG): $C_{26}H_{37}NO_7$ (475,26)

Beispiel 10

3-Benzyloxycarbonyl-4(S)-cyclohexylmethyl-2,2-dimethyl-5(S)-[1'-(2',2'-bismethoxycarbonyl-3'-methyl)butyl]-oxazolidin

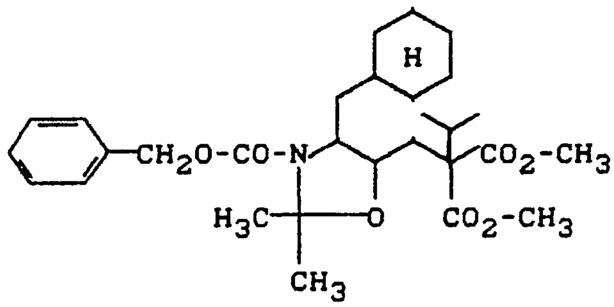

Unter Stickstoff werden 1,87 g (62,5 mmol) Natriumhydrid (80%ig) in 100 ml getrocknetem Tetrahydrofuran suspendiert. Dazu wird eine Lösung von 27 g (56,8 mmol) der Verbindung aus Beispiel 9 in 50 ml Tetrahydrofuran getropft. Nach 20 Minuten bei Rückflußtemperatur werden 56,81 ml (568 mmol) Isopropyljodid zugetropft. Die Lösung wird 12 h unter Rückfluß gekocht. Nach dem Abkühlen wird mit 1N Zitronensäure bis zur sauren Reaktion verrührt, das Lösemittel wird gegen Ethylacetat ausgetauscht. Nach Trocknen über Natriumsulfat und Einengen wird das Rohprodukt über eine Kieselgelsäule gereinigt [Petrolether (Sdp. 60-90° C) : Diethylether (1:1)1. Man erhält 25,94 g eines hellgelben Öls.

DC: $R_f$ (VII) = 0,59

DC: $R_f$ (VIII) = 0,48

MS (DCI) = 518

SF(MG): $C_{29}H_{43}NO_7$ (517,28)

Beispiel 11

3-Benzyloxycarbonyl-4(S)-cyclohexylmethyl-2,2-dimethyl-5(S)-[1'-(2'-carboxy-3'-methyl)butyl]oxazolidin

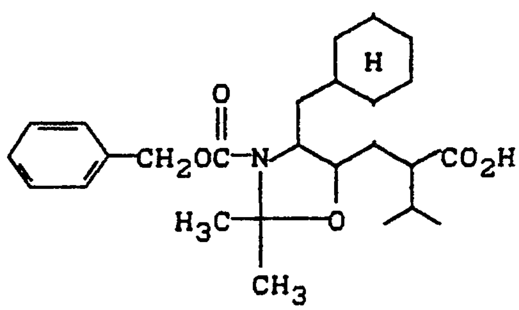

25,9 g (50,1 mmol) der Verbindung aus Beispiel 10 werden in 200 ml Methanol gelöst, mit 150,2 ml (300,4 mmol) 2N NaOH versetzt und 96 h am Rückfluß gekocht. Nach Abkühlen und Einengen wird der Rückstand in Wasser aufgenommen, 3 mal mit Diethylether und nach Ansäuern auf pH 2 wird 3 mal mit Ethylacetat extrahiert. Die Ethylacetatphase wird neutral gewaschen, getrocknet und eingeengt. Man erhält 21,3 g eines weißen Schaums.

DC: $R_f$ (IV) = 0,79

MS ($^e$FAB) = 444

SF(MG): $C_{26}H_{39}NO_5$ (445,6)

Beispiel 12

3-Benzyloxycarbonyl-4(S)-cyclohexylmethyl-2,2-dimethyl-5(s)-{1'-[2'-(carbonyl-S-isoleucinyl-(2-pyridylmethyl)-amidyl)-3'-methyl]butyl]}oxazolidin

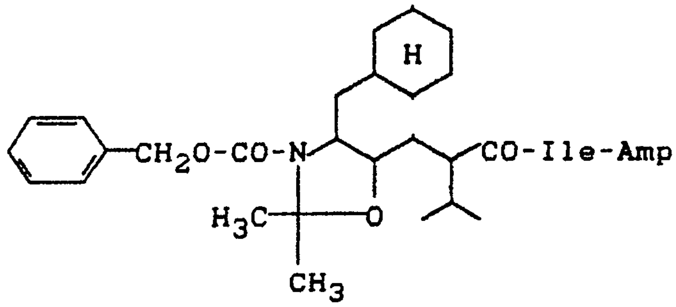

8 g (18 mmol) der Verbindung aus Beispiel 11 sowie 3,63 g (19 mmol) HOBT werden in 50 ml Dichlormethan gelöst und auf 0 °C gekühlt. 3,92 g (19 mmol) Dicyclohexylcarbodiimid werden in 30 ml Dichlormethan gelöst und zugegeben. Nach 1 h bei 0 °C ist Dicyclohexylharnstoff ausgefallen.

5,88 g (20 mmol) S-Isoleucinyl-2-pyridylmethylamid-dihydrochlorid werden in 50 ml Dichlormethan gelöst und mit 5,46 ml (45 mmol) N-Methylpiperidin versetzt. Nach 1 h unter Rühren wird die weiße Suspension zur obigen Suspension gegeben und 24 h gerührt. Die Reaktion wird durch Zugabe von 100 $\mu$l Eisessig gestoppt und das Lösemittel im Vakuum entfernt. Der Rückstand wird bei 0 °C mit Ethylacetat ausgerührt, der Harnstoff abgesaugt und nachgewaschen. Man erhält nach Trocknen über Natriumsulfat 12 g eines gelben Öls, das an Kieselgel chromatographiert wird [$CH_2Cl_2$/Methanol (95:5)]. Man erhält 10 g der Titelverbindung.

DC: $R_f$ (Ia) = 0,63
MS (FAB) = 649
SF(MG): $C_{38}H_{56}N_4O_5$ (648,4)

Beispiel 13

$N_\alpha$-{1-[5-(S}-Amino-6-cyclohexyl-4(S)-hydroxy-2-(1-methyl)ethyl]hexanoyl}-(S)-isoleucinyl-2-pyridylmethylamid

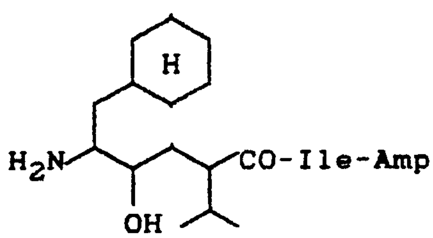

7 g (10,8 mmol) der Verbindung aus Beispiel 12 werden in 120 ml Methanol und 10 ml Eisessig gelöst und mit 200 mg Palladium auf Aktivkohle versetzt, Für 10 h wird ein Strom $H_2$ durchgeleitet, danach wird die Lösung über Kieselgur abgesaugt und mit 50 ml Kaliumhydrogensulfat-Lösung versetzt. Nach Abdestillieren des Methanols wird mit 80 ml Wasser verdünnt und zweimal mit Ethylacetat extrahiert. Die wässrige Lösung wird mit 2N NaOH auf pH 10 gebracht und dreimal mit Ethylacetat extrahiert. Nach Trocknen und Einengen der organischen Phase erhält man 3,2 g eines wachshaltigen Feststoffs.

DC: $R_f$ (IIIa) = 0,80
MS (FAB) = 475
SF(MG): $C_{27}H_{46}N_4O_3$ (474,3)

Beispiel 14

Nα-{Nδ-[Nα-(1,1-Dimethylethoxycarbonyl)-(S)-asparaginyl]-1-[5-S-amino-6-cyclohexyl-4-S-hydroxy-2-(1-methyl}-ethyl]hexanoyl}-(S)-isoleucinyl-2-pyridylmethylamid

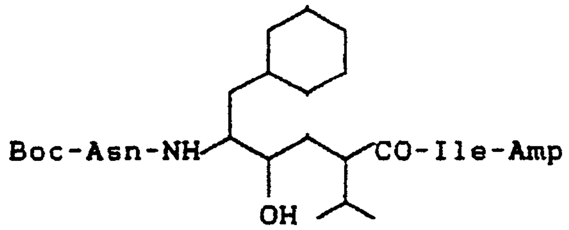

255 mg (1,1 mmol) Nα-1,1-Dimethylethoxycarbonylasparagin werden in 5 ml Dimethylformamid gelöst und mit 110 μl(1 mmol) N-Methylpiperidin versetzt. Nach Kühlung auf -20°C werden 136 mg (1 mmol) Chlorameisensäureisobutylester zugesetzt. Nach 15 Minuten Voraktivierung gibt man 474 mg (1 mmol) der Verbindung aus Beispiel 13, gelöst in 5 ml Dimethylformamid und 110 μl N-Methylpiperidin, langsam zu. Nach 3 h Rühren bei Raumtemperatur wird die Reaktion durch Zugabe von gesättigter Natriumhydrogencarbonatlösung gequenscht und eingeengt. Der Rückstand wird in Ethylacetat aufgenommen und zweimal mit gesättigter Natriumhydrogencarbonat-Lösung sowie mit Kochsalzlösung gewaschen, getrocknet und eingeengt. Nach HPLC (30% Acetonitril → 60% Acetonitril, 0,05% TFA) erhält man 10 mg der gewünschten Verbindung.
DC: $R_f$ (IIIa) = 0,90
MS (FAB) = 689
SF(MG): $C_{36}H_{60}N_6O_7$ (688,3)

Beispiel 15

Nα-{Nδ-[Nα-Phenylmethoxycarbonyl-(S)-asparaginyl]-1-[5-(S)-amino-6-cyclohexyl-4-(S)-hydroxy-2-(1-methyl)ethyl]-hexanoyl}-(S)-isoleucinyl-2-pyridylmethylamid

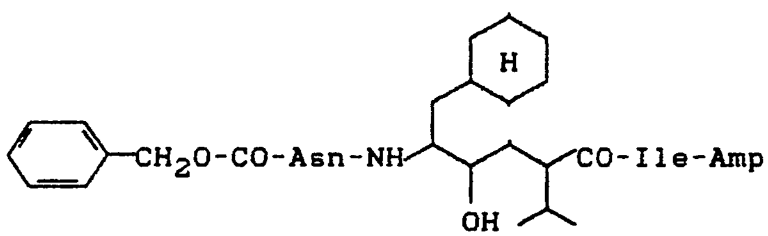

781 mg (2,93 mmol) Nα-Phenylmethoxycarbonyl-(S)-asparagin werden in 20 ml Dimethylformamid gelöst und mit 451 mg (2,93 mmol) 1-Hydroxybenzotriazol versetzte Nach Kühlung auf -10°C werden 1,24 g (2,93 mmol) N-Cyclohexyl-N'-(2-morpholinoethyl)-carbodiimid-metho-p-toluolsulfonat (Morpho-CDI) zugesetzt. 1,26 g (2,67 mmol) der Verbindung aus Beispiel 13 werden in 10 ml Dimethylformamid gelöst und mit 355 μl (2,93 mmol) N-Methylpiperidin versetzt und diese Lösung dann zu obiger Lösung zugetropft. Man rührt 12 h bei Raumtemperatur, engt ein und digeriert den Rückstand mit Wasser, dann mit Ethylacetat. Es werden 660 mg der Titelverbindung erhalten.
DC: $R_f$ (Ic) = 0,55
SF(MG): $C_{39}H_{58}N_6O_7$ (722,9)

Beispiel 16

Nα-{Nδ-[(S)-Asparaginyl]-1-[(5-(S)-amino-6-cyclohexyl-4-(S)-hydroxy-2-(1-methyl)ethyl]hexanoyl}-S-isoleucinyl-2-pyridylmethylamid

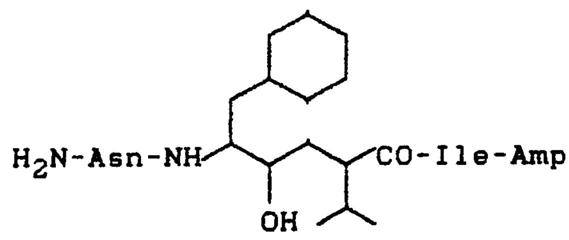

660 mg (0,914 mmol) der Verbindung aus Beispiel 15 werden in 50 ml Methanol gelöst und mit 100 mg Palladiuhydroxid versetzt. Nach 6 h $H_2$-Durchfluß wird über Kieselgur abgesaugt und eingeengt. Man erhält 600 mg der Titelverbindung.
DC: $R_f$ (Ic) = 0,44
MS (FAB) = 589
SF(MG): $C_{31}H_{52}N_6O_5$ (588.8)

Beispiel 17

Nα-{Nδ-{Nα-(Nα-Phenylmethoxycarbonyl-(S)-phenylalaninyl)-(S)-asparaginyl}-1-[5-(S)-amino-6-cyclohexyl-4-(S)-hydroxy-2-(1-methyl)ethyl]hexanoyl}-(S)-isoleucinyl-2-pyridylmethylamid

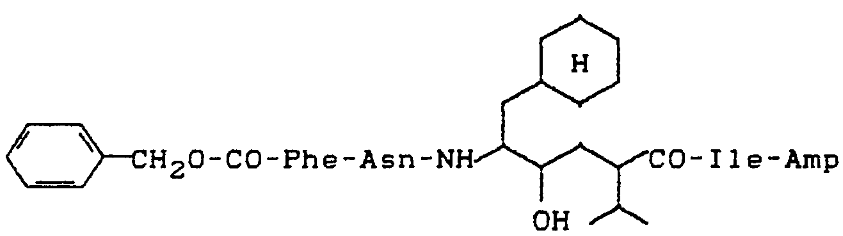

341,0 mg (1,15 mmol) N-Phenylmethoxycarbonyl-(S)-phenylalanin, 117,5 mg (1,14 mmol) 1-Hydroxybenzotriazol, 483,7 mg (1,142 mmol) Morpho-CDI, 537 mg (0,91 mmol) der Verbindung aus Beispiel 16 sowie 138 μl N-Methylpiperidin werden entsprechend Beispiel 15 zur Titelverbindung umgesetzt. Man erhält 540 mg.
DC: $R_f$ (Ic) = 0,58
SF(MG): $C_{48}H_{67}N_7O_8$ (870,1)

Beispiel 18

Nα-{Nδ-[Nα-((S)-Phenylalaninyl}-S-asparaginyl]-1-[5-(S)-amino-6-cyclohexyl-4-(S)-hydroxy-2-(1-methyl)-ethyl]hexanoyl}-S-isoleucinyl-2-pyridylmethylamid

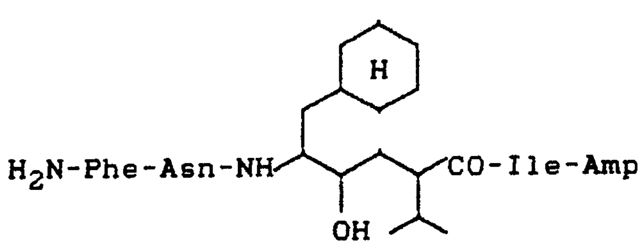

540 mg (62,1 mmol) der Verbindung aus Beispiel 17 werden in 50 ml Methanol gelöst und mit 100 mg Palladiumhydroxid versetzt. Nach 6 h Hydrierung bei Raumtemperatur wird über eine Filterschicht abgesaugt, zweimal mit heißem Methanol nachgewaschen und eingeengt. Man erhält nach Chromatographie über eine Kieselgelsäule [100% $CH_2Cl_2$ → $CH_2Cl_2/CH_3OH$ (93:7)] 371 mg der Titelverbindung.

DC: $R_f$ (Ic) = 0,58
MS (FAB+L) = 742
SF(MG): $C_{40}H_{61}N_7O_6$ (735,4)

Beispiel 19

Nα-{Nδ-{Nα-[Nα-(1,1-Dimethylethoxycarbonyl)-(S)-phenylalaninyl]-S-asparaginyl}-1-[5-(S)-amino-6-cyclohexyl-4-(S)-hydroxy-2-(1-methyl)ethyl]hexanoyl}-(S)-isoleucinyl-2-pyridylmethylamid

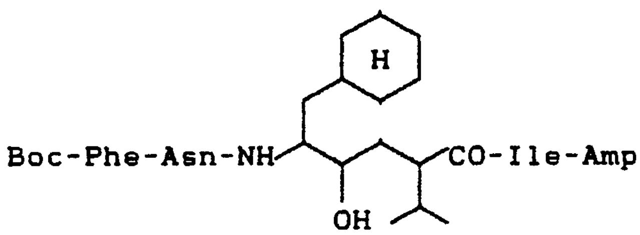

37 mg (0,05 mmol) der Verbindung aus Beispiel 18 werden in 5 ml Dimethylformamid gelöst und mit 30 mg (0,15 mmol) Bis(1,1-dimethylethyl)pyrocarbonat versetzt. Die Lösung wird mit einigen Tropfen Triethylamin alkalisch gemacht. Nach 2 h bei Raumtemperatur wird die Lösung eingeengt und mit Diethylether digeriert. Man erhält 10 mg der Titelverbindung.
DC; $R_f$ (IIIb) = 0,70
MS (FAB) = 836
SF(MG): $C_{45}H_{69}N_7O_8$ (835,4)

Beispiel 20

Nα-{Nδ-{Nα-{Nα-[Nα-[(1,1-Dimethylethoxycarbonyl)-(S)-seryl]-(S)-phenylalaninyl}-(S)-asparginyl}-1-1-[5-(S)-amino-6-cyclohexyl-4-(S)-hydroxy-2-(1-methyl)ethyl]-hexanoyl}-(S)-isoleucinyl-2-pyridylmethylamid

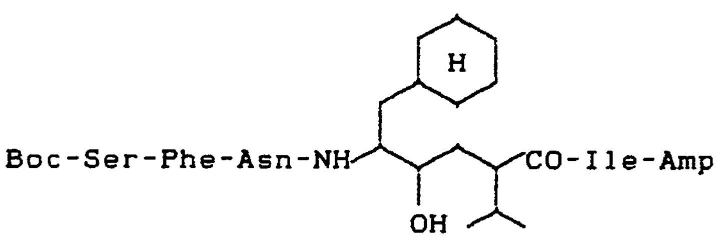

40 mg (192 μmol) Nα-(1,1-Dimethylethoxycarbonyl)-(S)-Serin, 31,1 mg (200 μmol) 1-Hydroxybenzotriazol, 85 mg (200 μmol) Morpho-CDI sowie 120 mg (160 μmol) der Verbindung aus Beispiel 18 werden in 8 ml Dimethylformamid entsprechend Beispiel 15 zur Titelverbindung umsetzt.
Ausbeute: 130 mg
DC: $R_f$ ($I_c$) = 0,63
MS (FAB) = 923
MS (FAB+Li) = 929
MS (FAB+Na) = 945
SF(MG): $C_{48}H_{74}N_8O_{10}$ (923,1)

Beispiel 21

Boc-AiB-Phe-Val-$OCH_3$

Eine gerührte, auf 0°C gekühlte Lösung von 10,17 g (50 0 mmol) 2-(1,1-Dimethylethoxycarbonyl)-amino-2-methyl-propionsäure und 7,09 g (52,5 mmol) HOBT in 140 ml wasserfreiem Dichlormethan wurde

mit 10.83 g (52.5 mmol) DCC versetzt. Das Kühlbad wurde entfernt und man rührte 30 min bei Raumtemperatur. Danach wurde erneut auf 0° C gekühlt, man gab eine Lösung von 17,47 g (55,5 mmol) HCl-H-Phe-Val-$OCH_3$ und 13,75 ml (125,0 mmol) N-Methylmorpholin in 140 ml Dichlormethan zu und rührte 15 h im auftauenden Eisbad nach. Der ausgefallene Harnstoff wurde durch Filtration abgetrennt, das Filtrat wurde mit 2 x 100 ml $NaHCO_3$-Lösung und 100 ml Wasser gewaschen und über $MgSO_4$ getrocknet. Nach Abdampfen des Lösungsmittels i.Vak. und Chromatographie des Rohprodukts an 270 g Kieselgel (Toluol:Ethylacetat 3:2) erhielt man 20.0 g (86 %) der Titelverbindung als farblosen Schaum.
DC: $R_f$ = 0,38 (Toluol:Ethylacetat 1:1)
MS (DCI, $NH_3$) m/Z = 464 $(M+H)^+$.
SF (MG): $C_{24}H_{37}N_3O_6$ (463.58)

Beispiel 22

Boc-AiB-Phe-Val-OH

Eine Lösung von 13.0 g (28.0 mmol) der Verbindung aus Beispiel 21 in 10 ml THF wurde mit einer Lösung von 2.35 g (56,0 mmol) Lithiumhydroxidhydrat in 55 ml Wasser versetzt und 3 h bei 0° C gerührt.

Danach goß man das Reaktionsgemisch in ein Gemisch aus 60 ml Wasser, 40 g Eis und 100 ml Ethylacetat und stellte durch Zugabe von 1 N Salzsäure pH 3 ein. Die organische Phase wurde abgetrennt, die Wasserphase mit 50 ml Ethylacetat extrahiert und die vereinigten organischen Extrakte wurden über Magnesiumsulfat getrocknet. Nach Abdampfen des Lösemitels im Vakuum und Behandeln des Rückstands mit 10 ml Ether und 30 ml n-Pentan erhielt man 10.3 g (82 % der Theorie) der Titelverbindung als farblose Kristalle.
Schmp.: 157° C
HPLC-Reinheit: > 96 %
DC:$R_f$ = 0,44 (Acetonitril:Wasser = 9,1).
MS (FAB): = m/z 350 $(M+H)^+$, 472 $(M+Na)^+$
SF (MG): $C_{23}H_{35}N_3O_6$ (449.55)

Beispiel 23

Boc-AiB-Val-$OCH_3$

Wie für Beispiel 21 beschrieben, erhielt man aus 10.17 g (50.0 mmol) 2-(1,1-Dimethylethoxycarbonyl)-amino-2-methyl-propionsäure und 9,19 g (55,5 mmol) HCl-H-Val-$OCH_3$ nach Chromatographie des Rohprodukts an 300 g Kieselgel (Toluol:Ethylacetat 1:1 10.3 g (79 %) der Titelverbindung als farblose Kristalle.
Schmp.: 108° C
DC:$R_f$ = 0,43 (Toluol;Ethylacetat 1:1)
MS (FAB) m/Z = 317 $(M+H)^+$.
SF (MG): $C_{15}H_{28}N_2O_5$ (316.40).

Beispiel 24

Boc-AiB-Val-OH

Wie für Beispiel 22 beschrieben, erhielt man aus 5,0 g (15,8 mmol) der Verbindung aus Beispiel 23 4,0 g (84 %) der Titelverbindung als farbloses Pulver.
Schmp.: 162° C
DC:$R_f$ = 0,5 (Acetonitril:Wasser 9:1)
MS (FAB) m/Z = 309 $(M+Li)^+$, 617 $(ZM+2Li-H)^+$.
SF (MG): $C_{14}H_{26}N_2O_5$ (302.38).

Beispiel 25

Boc-AiB-Ser-Phe-Val-$OCH_3$

Wie für Beispiel 21 beschrieben, erhielt man aus 104 mg (0,51 mmol) 2-(1,1-Dimethylethoxycarbonyl)-amino-2-methyl-propionsäure und 226 mg (0,56 mmol) HCl-H-Ser-Phe-Val-$OCH_3$ nach Chromatographie

des Rohprodukts an 40 g Kieselgel (Toluol:Ethylacetat 1:9) 203 mg (72 %) der Titelverbindung als helles Pulver.
Schmp.: ab 82° C (Zers.)
DC:$R_f$ = 0,24 (Ethylacetat)
MS (FAB) m/Z = 551 $(M+H)^+$, 573 $(M+Na)^+$.
SF(MG): $C_{27}H_{42}N_4O_8$ (550.66).

Beispiel 26

Boc-AiB-Ser-Phe-Val-OH

Wie für Beispiel 22 beschrieben, erhielt man aus 2.802 g (5,10 mmol) der Verbindung aus Beispiel 25 2.311 g (84 %) der Titelverbindung als helles Pulver.
Schmp.: ab 137° C (Zers.)
DC:$R_f$ = 0,31 (Acetonitril:Wasser 9:1)
MS (FAB) m/Z = 543 $(M=Li)^+$, 559 $(M+2Li)^+$.
SF (MG): $C_{26}H_{40}N_4O_8$ (536.64).

Beispiel 27

Boc-AiB-Ser-Phe-$OCH_3$

Wie für Beispiel 21 beschrieben, erhielt man aus 2,03 g (10,0 mmol) 2-(1,1-Dimethylethoxycarbonyl)-amino-2-methyl-propionsäure und 3,35 g (11,1 mmol) HCl-H-Ser-Phe-$OCH_3$ nach Chromatographie des Rohprodukts an 100 g Kieselgel (Ethylacetat) 3,92 mg %) der Titelverbindung als farblose Kristalle.
Schmp.: 123-125° C.
DC:$R_f$ = 0,31 (Ethylacetat)
MS (DCl, $NH_3$) m/Z = 452 $(M+H)^+$.
SF(MG): $C_{22}H_{33}N_3O_7$ (451.53).

Beispiel 28

Boc-AiB-Ser-Phe-OH

Wie für Beispiel 22 beschrieben, erhielt man aus 3,90 g (8,64 mmol) der Verbindung aus Beispiel 27 2.35 g (62 %) der Titelverbindung als hellen Schaum.
DC:$R_f$ = 0,25 (Acetonitril:Wasser 9:1)
MS (FAB) m/Z = 438 $(M+H)^+$, 460 $(M+Na)^+$.
SF (MG): $C_{21}H_{31}N_3O_7$ (437.50).

Beispiel 29

Boc-AiB-Pro-Phe-$OCH_3$

In Analogie zur Vorschrift des Beispiels 21 werden aus 5,6 g (27,6 mmol) 2-(1,1-Dimethyl-ethoxycarbonyl)amino-2-methylpropionsäure und 7,8 g (24,9 mmol) HCl•H-Pro-Phe-$OCH_3$ nach Chromatographie des Rohproduktes an 250 g Silicagel (Ethylacetat) 8,45 g (18,3 mmol) der Titelverbindung als farbloses Kristalle erhalten.
DC: $R_f$ (IIIa) = 0,87
MS(EI) m/Z = 462 $(M + H)^+$
SF (MG): $C_{24}H_{35}N_3O_6$ (461,56)

Beispiel 30

Boc-AiB-Pro-Phe-OH

In Analogie zur Vorschrift des Beispiels 22 werden aus 8,45 (18,2 mmol) der Verbindung aus Beispiel 29 8,0 g (17,9 mmol) der Titelverbindung als heller Schaum erhalten.

DC: $R_f$ (I) = 0,6
MS (FAB) m/Z: 448 $(M+H)^+$
SF (MG): $C_{23}H_{33}N_3O_6$ (447,53)

Beispiel 31

Nα-{Nδ-[Nα-(1,1-dimethylethoxycarbonyl)-Nα-methyl-Nπ-benzyloxymethyl-(S)-histidyl]-1-[5-(S)-amino-6-cyclohexyl-4-(S)-hydroxy-2-(1-methyl)ethyl]hexanoyl}-(S)-isoleucinyl-2-pyridylmethylamid

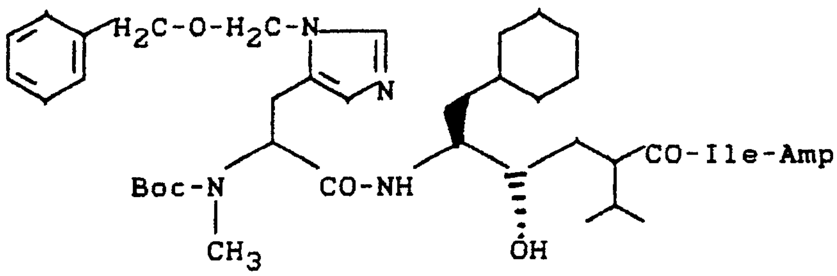

In Analogie zur Vorschrift des Beispiels 14 werden durch Umsetzung von 1,82 g (4,68 mmol) Boc-Nα-methyl-Nπ-Bom-(S)-histidin und der Verbindung aus Beispiel 13 nach Chromatographie des Rohprodukts an 100 g Silikagel 3,09 g (3,65 mmol) der Titelverbindung erhalten.
DC: $R_f$ (IIIb) = 0,62
MS(EI) m/Z = 846 $(M+H)^+$
SF (MG): $C_{47}H_{71}N_7O_7$ (845,53)

Beispiel 32

Nα-{Nδ-[Nα-(1,1-dimethylethoxycarbonyl)-Nα-methyl-(S)-histidyl]-1-[5-(S)-amino-6-cyclohexyl-4-(S)-hydroxy-2-(1-methyl)ethyl]-hexanoyl}-(S)-isoleucinyl-2-pyridylmethylamid

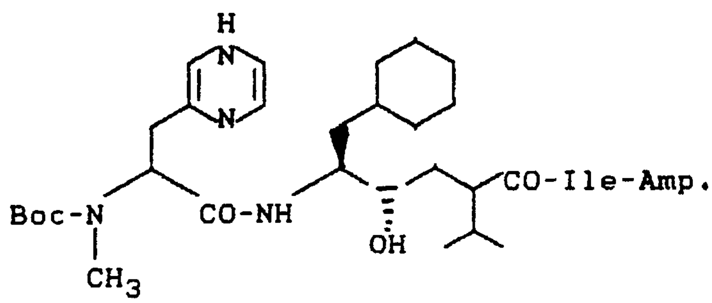

3,0 g (3,55 mmol) der Verbindung aus Beispiel 31 werden in 50 ml Methanol gelöst. Nach Zugabe von 4,53 g (72 mmol) Ammoniumformiat, 1,5 g Palladium/Aktivkohle und 300 µl (3,1 mmol) Triethylamin wird die Lösung 12 h unter Rückfluß gekocht. Nach Filtration wird das Lösemittel im Vakuum entfernt und das Rohprodukt mit Dichlormethan/Wasser extrahiert. Die organischen Phasen werden gesammelt, über Natriumsulfat getrocknet und bis zur Trockene im Vakuum eingeengt. Es werden 2,2 g (3 mmol) hellgelbe Kristalle der Titelverbindung erhalten.
DC $R_f$ (Ia) = 0,34
MS (FAB) m/Z = 726 $(M+H)^+$
SF (MG): $C_{39}H_{63}N_7O_6$ (725,45)

Beispiel 33

Nα-{Nδ-[Nα-methyl-(S)-histidyl]-1-[5-(S)-amino-6-cyclohexyl-4-(S)-hydroxy-2-(1-methyl)-ethyl]hexanoyl}-(S)-isoleucinyl-2-pyridylmethylamid

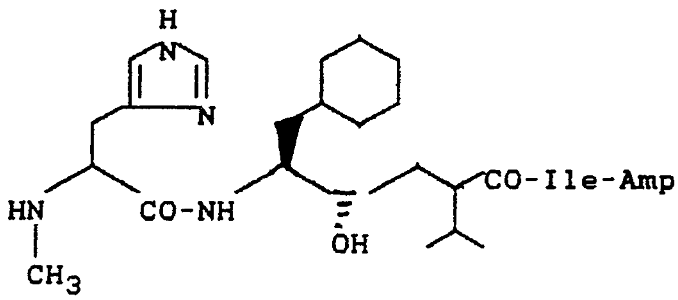

1,8 g (2,48 mmol) der Verbindung aus Beispiel 32 werden in Analogie zur Vorschrift des Beispiels 16 entschützt und gereinigt.
Man erhält 1,4 g (2,0 mmol) der Titelverbindung
DC $R_f$ (IIIb) = 0,44
MS (FAB) m/Z: 626 $(M+H)^+$
SF (MG): $C_{34}H_{55}N_7O_4 \cdot 2\ HCl$ (698,3)

Herstellungsbeispiele (allgemeine Formel I)

Beispiel I

Nα-{Nδ-{Nα-{1-[2-(1,1-Dimethylethoxycarbonyl)amino-2-methyl]propanoyl}-(S)-asparaginyl}-1-[5-(S)-amino-6-cyclohexyl-4-(S)-hydroxy-2-(1-methyl)ethyl]hexanoyl}-(S)-isoleucinyl-2-pyridylmethylamid

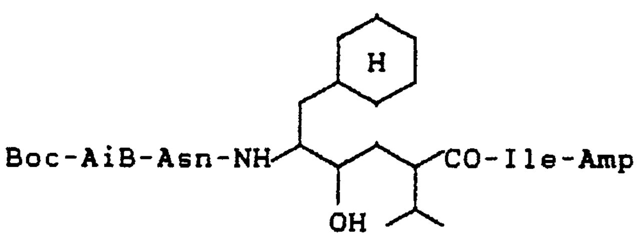

74,5 mg (367 μmol) 2-(1,1-Dimethylethoxycarbonyl)amino-2-methylpropionsäure und 58,35 mg (383 μmol) 1-Hydroxybenzotriazol werden in 5 ml Dimethylformamid gelöst und bei -10 °C 162 mg (383 μmol) Morpho-CDI zugegeben. Eine Lösung von 180 mg (306 μmol) der Verbindung aus Beispiel 16 und 46,3 μl N-methylpiperidin (383 μmol) werden in 5 ml Dimethylformamid gelöst. Beide Lösungen werden vereinigt und wie in Beispiel 15 gekuppelt und aufgearbeitet. Man erhält 150 mg der Titelverbindung.
DC: $R_f$ (IIIb) = 0,74
MS (FAB + Li) = 780
SF(MG): $C_{40}H_{67}N_7O_8$ (774,0)

Beispiel II

Nα-{Nδ-{Nα-{Nα-{1-[2-(1,1-Dimethylethoxycarbonyl)amino-2-methyl]propanoyl}-(S)-phenylalaninyl}-(S)-asparaginyl}-1-[5-(S)-amino-6-cyclohexyl-4-(S)-hydroxy-2-(1-methyl)ethyl]hexanoyl}-S-isoleucinyl-2-pyridylmethylamid

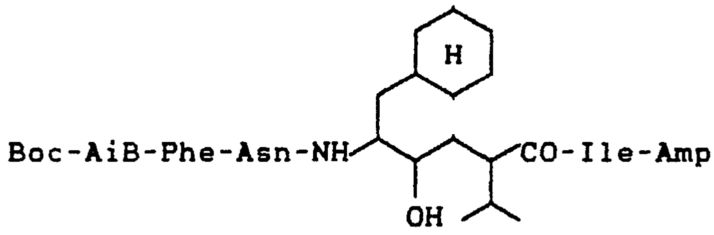

40 mg (192 μmol) 2-(1,1-Dimethylethoxycarbonyl)amino-2-methylpropionsäure, 31,1 mg (200 μmol) 1-Hydroxybenzotriazol, 120 mg (160 μmol) der Verbindung aus Beispiel 18 sowie 84,7 mg (200 μmol) Morpho-CDI werden in 9 ml Dimethylformamid entsprechend Beispiel 15 zur Titelverbindung umgesetzt. Man erhält 160 mg.
DC: $R_f$ (Ic) = 0,65
MS (FAB + Li) = 927
MS (FAB + Na) = 943
SF(MG): $C_{49}H_{76}N_8O_9$ (921,2)

Beispiel III

Nα-{Nδ-{Nα-{Nα-{Nα-{1-[2-(1,1-Dimethylethoxycarbonyl)amino-2-methyl]-propanoyl}-(S)-seryl}-(S)-phenylalaninyl}-(S)-asparaginyl}-[1-(5-(S)-amino-6-cyclohexyl-4-(S)-hydroxy-2-(1-methyl)ethyl]hexanoyl}-(S)-isoleucinyl-2-pyridylmethylamid

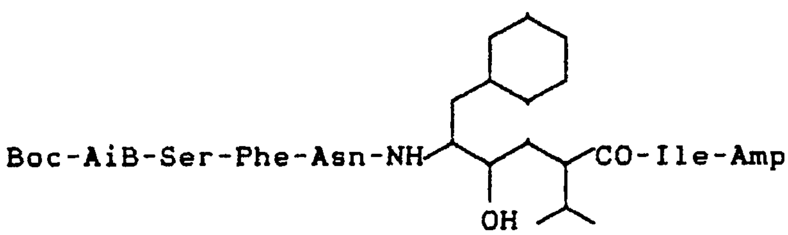

55,7 mg (192 μmol) Nα-[2-(1,1-Dimethylethoxycarbonyl)-amino-2-methylpropanoyl]-(S)-Serin, 31,1 mg (200 μmol) 1-Hydroxy-benzotriazol, 84,7 mg (200 μmol) Morpho-CDI sowie 120 mg (100 μmol) der Verbindung aus Beispiel 18 werden in 9 ml Dimethylformamid entsprechend Beispiel 15 zur Titelverbindung umgesetzt.
Ausbeute: 150 mg
DC: $R_f$ (Ic) = 0,54
MS (FAB) = 1008
MS (FAB + Na) = 1030
SF(MG): $C_{52}H_{81}N_9O_{11}$ (1008,3)

Beispiel IV

Nα-{Nδ-{1-[2-(1,1-Dimethylethoxycarbonyl)amino-2-methyl]-propanoyl}-1-[5-(S)-amino-6-cyclohexyl-4-(S)-hydroxy-2-(1-methyl)ethyl]hexanoyl}-(S)-isoleucinyl-2-pyridylmethylamid

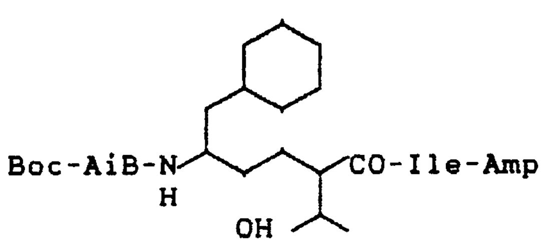

Eine gerührte, auf 0°C gekühlte Lösung von 43,5 mg (0,214 mmol) 2-(1,1-Dimethylethoxycarbonyl)-amino-2-methylpropionsäure und 33,2 mg (0,246 mmol) HOBT in 1 ml wasserfreiem Dichlormethan wurde mit 48 mg (0,232 mmol) DCC versetzt, Nach ca. 5 min gab man eine Lösung von 109,4 mg (0,20 mmol) der Verbindung aus Beispiel 13 und 77 µl (0,70 mmol) N-Methylmorpholin in 1 ml Dichlormethan zu, entfernte das Kühlbad und rührte 18 h bei Raumtemperatur nach. Der ausgefallene Harnstoff wurde durch Filtration abgetrennt, das Filtrat wurde im Vakuum eingeengt und an 15 g Kieselgel (Ethylacetat) chromatographiert. Man erhielt 67 mg (51 %) der Titelverbindung als farblose Kristalle; Diastereomerengemisch.

Schmp.: 127-8°C.

DC:$R_f$ = 0,19 (Ethylacetat)

MS (FAB) m/Z = 660 $(M+H)^+$.

SF(MG): $C_{36}H_{61}N_5O_6$ (659.92).

Tabelle 1

Wie für Beispiel IV beschrieben erhielt man durch Umsetzung der Verbindung aus Beispiel 13 und der entsprechenden Säure (Ausgangsverbindung) die folgenden Produkte:

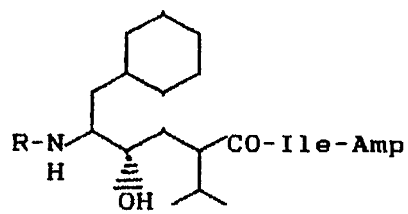

| Bsp. Nr. | R | Ausbeute (%) | MS(FAB) m/Z $(M+H)^+$ | $R_f$/Laufmittel (Verhältnis) | Ausgangsverbindung aus Beispiel |
|---|---|---|---|---|---|
| V | Boc-Aib-Val | 48 | 759 | 0,15 (Ethylacetat)[a] | 24 |
| VI | a Boc-Aib-Phe-Val | 28 | 906 | 0,29 (Ia) unpolar[b] | 22 |
| | b Boc-Aib-Phe-Val | 12 | 906 | 0,26 (Ia) polar[b] | |
| VII | a Boc-Aib-Ser-Phe-Val | 12 | 993 | 0,23 (Ia) unpolar | 26 |
| | b Boc-Aib-Ser-Phe-Val | 11 | 993 | 0,19 (Ia) polar | |
| VIII | a Boc-Aib-Ser-Phe | 23 | 912 | 0,20 (Ia) unpolar | 28 |
| | b Boc-Aib-Ser-Phe | | 912 | 0,17 (Ia) polar | |

a Gemisch der $Pr^i$-Diastereomere

b reine Diastereomere

Beispiel IX

Nα-{Nδ-{Nα-{Nα-{Nα-{1-[2-(1,1-dimethyl-ethoxycarbonyl)amino-2-methyl]-propanoyl]-(S)prolyl}-(S)-phenylalaninyl}-(S)-Nα-methyl-histidyl}-1-[5-(S)-amino-6-cyclohexyl-4-(S)-hydroxy-2-(1-methyl)-ethyl]-

hexanoyl}-(S)-isoleucinyl-2-pyridylmethylamid

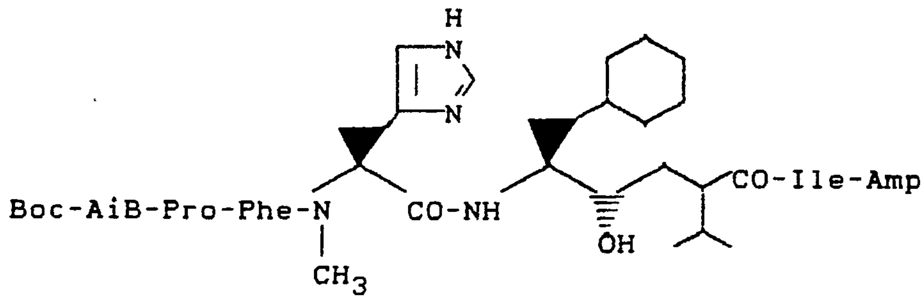

In Analogie zur Vorschrift des Beispiels IV wird durch Umsetzung der Verbindungen aus den Beispielen 30 (Säure) und 33 (Aminkomponente) hergestellt.
Ausbeute: 26 %
DC $R_f$ (Ic) = 0,78
MS (FAB): 1055
SF (MG): $C_{57}H_{86}N_{10}O_9$ (1054,65)

Beispiel X

Nα-{Nδ-{Nα-{Nα-{Nα-{1-[2-acetylamino-2-methyl]-propanoyl-}-(S)-propyl}-(S)-phenylalanyl}-(S)-Nα-methyl-histidyl]-1-[5-(S)-amino-6-cyclohexyl-4-(S)-hydroxyl-2-(1-methyl)ethyl]hexanoyl}-(S)-isoleucinyl-2-pyridylmethylamid

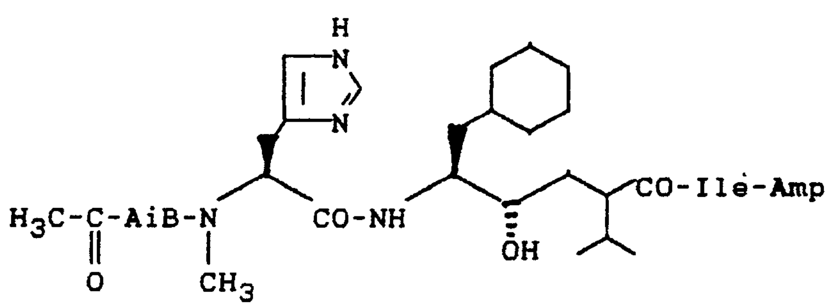

135 mg 2-Acetylamino-2-methylpropionsäure (0,93 mmol) werden in 5 ml Acetonitril und 1 ml DMF gelöst. Die Säure wird mit 412 mg (0,93 mmol) BOP voraktiviert, 500 mg (0,62 mmol) der Verbindung aus Beispiel 33 werden in 3 ml Acetonitril gelöst; danach werden 75 µl (0,62 mmol) N-methylpiperidin zugegeben. Nach 30' werden die Lösungen zusammengegeben und 24 h gerührt. Nach Entfernung des Lösemittels wird der Rückstand zwischen Ethylacetat und Natriumhydrogencarbonat aufgenommen. Die organischen Phasen werden nacheinander mit Natriumhydrogencarbonat und Hochsalzlösung gewaschen, über Natriumsulfat getrocknet und eingedampft.

Nach Präp. HPLC, Dynamax RP-18, Gradient 10 % auf 90 % Acetonitril, erhält man 165 mg eines weißen, flockigen Lyophylisats.
HPCL: (Deltapak 7 µ, 100 Å) Gradient 10 % auf 90 %
Acetonitril in 15' : $R_+$ = 10,8
MS (FAB) m/Z = 753 $(M+H)^+$
SF (MG): $C_{40}H_{64}N_8O_6$ (753,00)

**Patentansprüche**

1. Peptide der allgemeinen Formel (I)

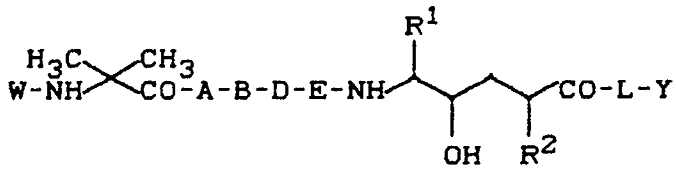

(I)

in welcher

W

- für eine Aminoschutzgruppe steht, oder
- für eine Gruppe der Formel

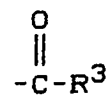

steht,

worin

$R^3$

- Wasserstoff, geradkettiges oder verzweigtes Alkyl mit bis zu 8 Kohlenstoffatomen oder Aryl mit 6 bis 10 Kohlenstoffatomen bedeutet, das seinerseits durch Halogen, Hydroxy, Nitro, Trifluormethyl oder durch geradkettiges oder verzweigtes Alkyl oder Alkoxy mit bis zu 8 Kohlenstoffatomen substituiert sein kann,

A, B, D, E und L gleich oder verschieden sind und

- für eine direkte Bindung oder
- für einen Rest der Formel

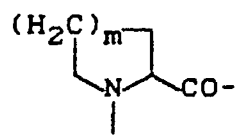

stehen

worin

m

- die Zahl 1 oder 2 bedeutet,

oder

- für eine Gruppe der Formel

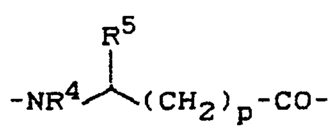

stehen

worin

p

- die Zahl 0, 1 oder 2 bedeutet,

$R^4$

- Wasserstoff, geradkettiges oder verzweigtes Alkyl mit bis zu 8 Kohlenstoffatomen oder Phenyl bedeutet,

$R^5$

- Cycloalkyl mit 3 bis 8 Kohlenstoffatomen oder Aryl mit 6 bis 10 Kohlenstoffatomen bedeutet, oder
- Wasserstoff oder geradkettiges oder verzweigtes Alkyl mit bis zu 8 Kohlenstoffatomen

bedeutet,
das gegebenenfalls durch Cycloalkyl mit 3 bis 8 Kohlenstoffatomen oder durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe $-NR^6R^7$ substituiert ist,
oder das gegebenenfalls durch einen 5-oder 6-gliedrigen stickstoffhaltigen Heterocyclus oder Indolyl substituiert ist, worin die entsprechenden -NH-Funktionen gegebenenfalls durch Alkyl mit bis zu 6 Kohlenstoffatomen oder durch eine Aminoschutzgruppe geschützt sind,
oder das gegebenenfalls durch Alkylthio mit bis zu 6 Kohlenstoffatomen, Hydroxy, Mercapto, Guanidyl oder durch eine Gruppe der Formel $-NR^6R^7$ oder $R^8OC$-substituiert ist,
worin
$R^6$ und $R^7$
gleich oder verschieden sind und Wasserstoff, geradkettiges oder verzweigtes Alkyl mit bis zu 8 Kohlenstoffatomen oder Phenyl bedeuten
und
$R^8$
- Hydroxy, Benzyloxy, Alkoxy mit bis zu 6 Kohlenstoffatomen oder die oben aufgeführte Gruppe $-NR^6R^7$ bedeutet,

in ihrer D- oder L-Form, oder als D,L-Isomerengemisch, bevorzugt in der L-Form,
$R^1$ und $R^2$ gleich oder verschieden sind und
- für Cycloalkyl mit 3 bis 8 Kohlenstoffatomen stehen,
- für geradkettiges oder verzweigtes Alkyl oder Alkenyl mit bis zu 10 Kohlenstoffatomen stehen, die gegebenenfalls durch Cycloalkyl mit 3 bis 8 Kohlenstoffatomen oder Aryl mit 6 bis 10 Kohlenstoffatomen substituiert sind, das seinerseits bis zu 3-fach gleich oder verschieden durch Halogen, Cyano, Nitro oder durch eine Gruppe der Formel $-OR^9$ substituiert sein kann,
  worin
  $R^9$
  - Wasserstoff oder eine typische Hydroxyschutzgruppe bedeutet,

Y
- für eine Gruppe der Formel $-NHR^{10}$ steht, worin
  $R^{10}$
  Cycloalkyl mit 3 bis 8 Kohlenstoffatomen bedeutet, oder
  - geradkettiges oder verzweigtes Alkyl mit bis zu 8 Kohlenstoffatomen bedeutet, das gegebenenfalls durch Pyridyl oder Phenyl substituiert ist

und deren physiologisch unbedenklichen Salze.

**2.** Peptide der allgemeinen Formel (I) gemäß Anspruch 1
in welcher
W
- für eine der oben aufgeführten Aminoschutzgruppen steht, vorzugsweise für tert.Butyloxycarbonyl (Boc), 9-Fluorenylmethyloxycarbonyl (Fmoc) oder Benzyloxycarbonyl (Z), oder
- für eine Gruppe der Formel

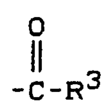

  steht,
  worin
  $R^3$
  - Wasserstoff, geradkettiges oder verzweigtes Alkyl mit bis zu 6 Kohlenstoffatomen oder Phenyl bedeutet, das seinerseits durch Fluor, Chlor, Brom oder durch geradkettiges oder verzweigtes Alkyl oder Alkoxy mit bis zu 6 Kohlenstoffatomen substituiert sein kann,

A, B, D, E und L gleich oder verschieden sind und
- für eine direkte Bindung oder
- für Prolin stehen, oder
- für eine Gruppe der Formel

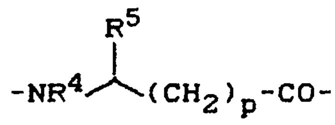

stehen
worin
p
die Zahl 0 oder 1 bedeutet,
$R^4$
- Wasserstoff oder geradkettiges oder verzweigtes Alkyl mit bis zu 6 Kohlenstoffatomen oder Phenyl bedeutet,

$R^5$
- Cyclopentyl oder Cyclohexyl bedeutet, oder Wasserstoff, Phenyl oder geradkettiges oder verzweigtes Alkyl mit bis zu 6 Kohlenstoffatomen bedeutet, das gegebenenfalls durch Hydroxy, HO-CO- oder $H_2N$-CO- substituiert sein kann,
  oder durch Cyclohexyl oder Naphthyl oder Phenyl substituiert ist, die ihrerseits durch Fluor, Chlor, Nitro oder Alkoxy mit bis zu 6

Kohlenstoffatomen substituiert sein können,
oder durch Indolyl, Imidazolyl, Pyridyl, Triazolyl oder Pyrazolyl substituiert ist, wobei die entsprechenden -NH-Funktionen gegebenenfalls durch Alkyl mit bis zu 4 Kohlenstoffatomen oder durch eine Aminoschutzgruppe geschützt sind,

in ihrer D- oder L-Form, oder als D,L-Isomerengemisch, bevorzugt in ihrer L-Form, $R^1$ und $R^2$ gleich oder verschieden sind und
- für Cyclopropyl, Cyclopentyl oder Cyclohexyl stehen,
- für geradkettiges oder verzweigtes Alkyl oder Alkenyl mit bis zu 8 Kohlenstoffatomen stehen, die gegebenenfalls durch Cyclopropyl, Cyclobutyl, Cyclopentyl, Cyclohexyl, Cycloheptyl oder Phenyl substiutiert sind, das seinerseits bis zu 2-fach gleich oder verschieden durch Fluor, Chlor, Brom, Cyano, Nitro oder durch eine Gruppe der Formel $-OR^9$ substiutiert ist,
  worin
  $R^9$
  - Wasserstoff, tert.Butyl oder Benzyl bedeutet,

Y
- für eine Gruppe der Formel $-NHR^{10}$ steht,
  worin
  $R^{10}$
  geradkettiges oder verzweigtes Alkyl mit bis zu 6 Kohlenstoffatomen bedeutet, das gegebenenfalls durch Cyclopropyl, Cyclopentyl, Cyclohexyl, Phenyl oder Pyridyl substituiert ist

und deren physiologisch unbedenklichen Salze.

**3.** Peptide der allgemeinen Formel (I) gemäß Anspruch 1
in welcher
W
- für die Aminoschutzgruppen Boc, FMoc oder Benzyloxycarbonyl steht, oder
- für eine Gruppe der Formel

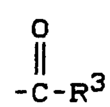

steht,
worin
$R^3$
- Wasserstoff, geradkettiges oder verzweigtes Alkyl mit bis zu 4 Kohlenstoffatomen oder Phenyl bedeutet,

A, B, D, E und L gleich oder verschieden sind und

- für eine direkte Bindung oder
- für Prolin stehen, oder
- für eine Gruppe der Formel

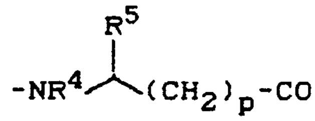

stehen,
worin
p
die Zahl 0 oder 1 bedeutet,
$R^4$
- Wasserstoff oder Methyl bedeutet,
$R^5$
- Cyclopentyl oder geradkettiges oder verzweigtes Alkyl mit bis zu 4 Kohlenstoffatomen bedeuten, das gegebenenfalls durch Hydroxy, HO-CO- oder $H_2N$-CO- substituiert ist, oder durch Cyclohexyl oder Naphthyl oder Phenyl substituiert ist, die ihrerseits durch Fluor, Chlor oder Alkoxy mit bis zu 4 Kohlenstoffatomen substituiert sein können, oder durch Imidazolyl, Triazolyl, Pyridyl oder Pyrazolyl substituiert ist, wobei die NH-Funktion gegebenenfalls durch Methyl, Benzyloxymethylen oder t-Butyloxycarbonyl (Boc) geschützt sind,

in ihrer D- oder L-Form oder als D,L-Isomerengemisch, bevorzugt in der L-Form,
$R^1$ und $R^2$ gleich oder verschieden sind und
- für Cyclopropyl, Cyclopentyl oder Cyclohexyl stehen, oder
- für geradkettiges oder verzweigtes Alkyl oder Alkenyl mit bis zu 6 Kohlenstoffatomen stehen, die gegebenenfalls durch Cyclopropyl, Cyclopentyl, Cyclohexyl oder Phenyl substituiert sind, das seinerseits durch eine Gruppe der Formel $-OR^9$ substituiert ist,
worin
$R^9$
- Wasserstoff, tert.Butyl oder Benzyl bedeutet,

Y
- für eine Gruppe der Formel $-NHR^{10}$ steht,
worin
$R^{10}$
geradkettiges oder verzweigtes Alkyl mit bis zu 4 Kohlenstoffatomen bedeutet, das gegebenenfalls durch Cyclopropyl, Cyclohexyl, Phenyl oder Pyridyl substituiert ist

und deren physiologisch unbedenklichen Salze.

**4.** Verfahren zur Herstellung der Verbindungen gemäß Ansprüchen 1-3, dadurch gekennzeichnet, daß man Verbindungen der allgemeinen Formel (II)

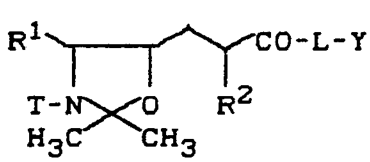

(II)

in welcher
$R^1$, $R^2$ L und Y die oben angegebene Bedeutung haben
und
T
- für eine der oben aufgeführten Aminoschutzgruppen, vorzugsweise für Benzyloxycarbonyl steht,

in inerten Lösemitteln zu den Aminoalkoholen der Formel (III)

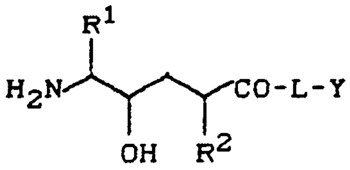

(III)

in welcher
$R^1$, $R^2$, L und Y die oben angegebene Bedeutung haben,
unter Ringöffnung reduziert, und anschließend mit Verbindungen der Formel (IV)

T'-E'-OH (IV)

in welcher
T'
- die oben angegebene Bedeutung von T hat und mit dieser gleich oder verschieden ist

und
E'
die oben angegebene Bedeutung von E hat, aber nicht für eine direkte Bindung steht, nach üblicher Methode kondensiert. in einem nächsten Schritt, entweder zunächst die Schutzgruppe T' abspaltet, erneut deblockiert und mit Verbindungen der allgemeinen Formel (V),

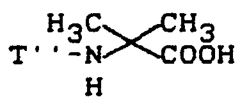

(V)

in welcher
T''
die oben angegebene Bedeutung von T hat und mit dieser gleich oder verschieden ist,
gegebenenfalls unter Aktivierung der Carbonsäure nach üblicher Methode zu Verbindungen der allgemeinen Formel (Ia)

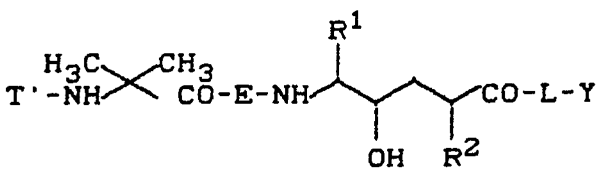

(Ia)

in welcher
T', E, $R^1$, $R^2$, L und Y die oben angegebene Bedeutung haben,
umsetzt oder zunächst die Aminosäuregruppierungen B und D in Analogie zu der oben beschriebenen Umsetzung mit den Verbindungen der Formel (IV) unter Aktivierung und Deblockierung einführt und in einem letzten Schritt mit Verbindungen der allgemeinen Formel (VI)

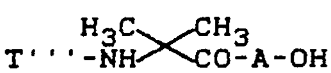

(VI)

in welcher
A die oben angegebene Bedeutung hat
und
T'''
die oben angegebene Bedeutung von T hat und mit dieser gleich oder verschieden ist,
umsetzt.

5. Arzneimittel enthaltend mindestens eine Verbindung gemäß den Ansprüchen 1 bis 3.

6. Verwendung der Verbindungen gemäß den Ansprüchen 1 bis 3 bei der Bekämpfung von Krankheiten.